# FASCICULE DE BREVET EUROPEEN

(11) **EP 0 751 162 B1**
(45) Date de publication et mention de la délivrance du brevet: **29.10.1997**
(21) Numéro de dépôt: 96401141.5
(22) Date de dépôt: 28.05.1996
(51) Int. Cl.: C08G 18/61, A61K 7/48, A61K 7/06

(54) **Utilisation dans et pour la fabrication de compositions cosmétiques ou dermatologiques de polycondensats séquencés polyuréthanes et/ou polyurées à greffons siliconés**
Verwendung in und für die Herstellung von kosmetischen oder dermatologischen Zusammensetzungen von Blockpolykondensaten aus Polyurethanen und/oder Polyharnstoffen mit Selikon-Pfropfungen
Use in and for the preparation of cosmetic or dermatological compositions from polyurethane and/or polyurea blockpolycondensates having silicone grafts

(30) Priorité: 27.06.1995 FR 9507731
(43) Date de publication de la demande: 02.01.1997
(73) Titulaire: L'OREAL, 75008 Paris (FR)
(72) Inventeur: Mougin, Nathalie, 75011 Paris (FR); Mondet, Jean, 93600 Aulnay-sous-Bois (FR)
(74) Mandataire: Miszputen, Laurent

(56) Documents cités:
- EP-A- 0 277 816
- EP-A- 0 324 946
- EP-A- 0 636 361
- FR-A- 2 645 156

## Description

La présente invention concerne l'utilisation dans et pour la fabrication de compositions cosmétiques ou dermatologiques, notamment dans le domaine capillaire, du maquillage et des produits de soin ou d'hygiène de polycondensats séquencés polyuréthanes et/ou polyurées comportant une chaîne constituée par la répétition d'au moins une séquence polyuréthane et/ou polyurée -[-M-]- comportant un greffon polysiloxane.

Il est d'usage courant de mettre en oeuvre dans des formulations cosmétiques des polymères organiques filmogènes pour apporter des propriétés de maintien et de fixation des cheveux dans les produits de coiffage rincés et non rincés, des propriétés de gainage des cils dans les mascaras, des propriétés de revêtement protecteur dans les vernis à ongles ou les produits de soin de la peau ainsi que des propriétés d'adhésion dans les produits de maquillage tels que les poudres compactées, les fonds de teint, les eye-liners ou les produits de soin de la peau.

Ces différentes propriétés sont généralement liées aux caractéristiques filmogènes du polymère organique en particulier les propriétés mécaniques et l'énergie de surface (adhésion).

Il est également d'usage courant d'utiliser des silicones sous forme d'huiles, de gommes ou de cires pour apporter en plus, aux matières kératiniques traitées, des propriétés de surface telles que la brillance dans le domaine des produits capillaire, des mascaras ou des vernis à ongles, la lubrification, la rémanence vis à vis de l'eau, la douceur au toucher sans aspect gras. L'utilisation de silicones plus ou moins fluides permet d'obtenir ces propriétés mais leur dépôt sur les cheveux, les ongles, les cils ou la peau n'est pas durable du fait qu'ils présentent des propriétés mécaniques insuffisantes.

Ces dernières années, un intérêt tout particulier s'est manifesté pour la réalisation de compositions cosmétiques filmogènes contenant l'association d'un ou plusieurs polymères organiques filmogènes avec une ou plusieurs silicones.

Cependant, ces associations n'apportent pas l'additivité des propriétés spécifiques à chaque constituant de ces associations.

En effet, les silicones les plus performantes à savoir les polydiméthylsiloxanes et les polyméthylphénylsiloxanes sont incompatibles avec la plupart des polymères organiques dans la formulation. D'autre part, pour que ces associations puissent apporter les propriétés attendues, il faut que les mélanges de ces constituants conduisent à une stratification après application et séchage. Le polymère filmogène doit aller au contact direct avec le cheveu, le cil, l'ongle ou la peau et apporter une bonne adhésion tandis que la silicone doit, de préférence, aller en surface pour y apporter ses caractéristiques de brillance, de lubrification, de toucher doux et de rémanence vis-à-vis de l'eau. Il est donc difficile, par simple mélange des polymères filmogènes et des silicones dans une formulation, d'obtenir à la fois une bonne compatibilité des constituants et l'additivité de leurs propriétés.

Pour surmonter ces inconvénients, on connaît des polycondensats multiséquencés dont la chaîne est constituée d'au moins une séquence polyuréthane et/ou polyurée et comportant des groupements anioniques ou cationiques et d'au moins une séquence polysiloxane. Ils sont décrits dans la demande de brevet EP-A-0 636 361. Pour obtenir de bonnes propriétés de surface spécifiques aux silicones, ces copolymères multiséquencés doivent comporter une quantité importante de séquences polysiloxanes dans la chaîne qui a pour inconvénient de diminuer les propriétés filmogènes et notamment mécaniques recherchées pour les diverses applications cosmétiques (films trop mous ou élastomères). D'autre part, ce type de copolymère n'est utilisé qu'en dispersion aqueuse sous forme de pseudo-latex; ce qui limite considérablement les possibilités de les utiliser dans d'autres formes de formulations telles que des solutions organiques ou hydroorganiques et notamment de préparer des formulations anhydres comme des rouges à lèvres, des mascaras water-proof, des poudres de maquillage.

La demanderesse a découvert de manière surprenante une nouvelle famille de polycondensats séquencés dont la chaîne est constituée par la répétition d'au moins une séquence polyuréthane et/ou polyurée comportant un greffon polysiloxane.

Les polymères selon la présente invention, apportent de bonnes caractéristiques d'adhésion sur les cheveux, les cils, les ongles et la peau. Ils permettent d'obtenir des dépôts ou revêtements très rémanents à l'action de l'eau ou de l'eau en présence de tensio-actifs. Ces dépôts présentent en outre en surface les caractéristiques des silicones à savoir la brillance, la lubrification, la rémanence, la douceur au toucher. Ces dépôts peuvent conserver des propriétés mécaniques interessantes lorsque les polymères de l'invention sont utilisés seuls ou en association avec d'autres polymères organiques.

Les polycondensats polyuréthanes et/ou polyurées à greffons siliconés de l'invention permettent d'obtenir une bonne additivité des propriétés filmogènes des polyuréthanes et/ou polyurées et des caractéristiques de surface des silicones.

Ils présentent également par rapport aux polycondensats polyuréthane et/ou polyurées à séquences siliconées de l'art antérieur, des propriétés de surface conférées par les silicones sensiblement améliorées telles que la brillance, la lubrification, le toucher doux, la rémanence vis-à-vis de l'eau sans affecter les propriétés filmogènes du polymère.

Les polycondensats séquencés selon l'invention présentent l'avantage d'être compatibles avec des silicones traditionnelles et/ou d'autres polymères organiques additionnels présents également dans une même formulation. Ils permettent par leur chaîne greffée par des silicones de rendre compatibles ces silicones classiques en association avec ces polymères organiques additionnels.

Ils peuvent être de nature anionique, cationique, amphotère ou non-ionique et sont insolubles dans l'eau. Leur structure permet non seulement leur utilisation dans des dispersions aqueuses sous forme de pseudo-latex dont la définition sera donnée plus loin mais permet également leur utilisation en solution dans un solvant organique ; ce qui donne lieu à de plus grandes possibilités de formulations cosmétiques.

Les polycondensats séquencés polyuréthanes et/ou polyurées selon l'invention sont caractérisées par le fait que leur chaîne est constituée par la répétition d'au moins une séquence -[-M-]- polyuréthane et/ou polyurée comportant un greffon polysiloxane.

Les poids moléculaires moyens en nombre des polycondensats polyuréthanes et/ou polyurées greffés siliconés peuvent varier dans de larges limites, de préférence entre 2.000 et 500.000 mais plus préférentiellement entre 3.000 et 250.000.

Les polycondensats selon l'invention sont préférentiellement multiséquencés et leur chaîne est constitué également par la répétition d'une séquence -[-N-]- de polyuréthane et/ou polyurée comportant des groupes non-ioniques et/ou des groupes anioniques, cationiques ou amphotères et/ou par la répétition d'une séquence -[-G-]- de polyuréthane et/ou de polyurée comportant des oligomères de polymères organiques.

Les polycondensats selon l'invention peuvent également comporter dans leur chaîne la répétition d'une séquence polysiloxane-[-L-]-.

Les différentes séquences -[-M-]-, -[-N-]-, -[-G-]- et -[-L-]- constituant la chaîne des polycondensats multiséquencés de l'invention sont réparties, de préférence, de façon aléatoire.

Pour une bonne compréhension de l'exposé qui va suivre, et en particulier de la définition des formules données, on commencera par expliquer, mais seulement dans ses grandes lignes directrices, le procédé général de synthèse des polycondensats séquencés de l'invention. Les détails du procédé seront données plus loin. De même, seront précisés ultérieurement les significations et valeurs de certains radicaux et autres paramètres, qui n'apparaîtront donc dans la partie introductrice donnée maintenant qu'à titre symbolique et par commodité.

Les polycondensats conformes à l'invention peuvent être préparés selon un procédé comportant au moins une étape de réaction classique de polycondensation entre (i) un oligomère polysiloxane présentant une fonction diol ou diamine sur une seule extrémité et de formule : 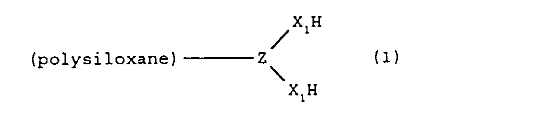 et (ii) un diisocyanate de formule :

O=C=N-R-N=C=O (2).

On obtient un polycondensat constitué par la répétition d'une séquence polyuréthane et/ou polyurée greffée par un polysiloxane répondant à la formule :

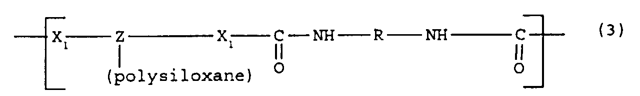

Dans les formules (1), (2) et (3), X₁ représente, séparément ou conjointement, -O- ou -NH- et Z un radical trivalent hydrocarboné pouvant contenir un ou plusieurs hétéroatomes tels que l'oxygène, le souffre, l'azote.

Si l'on souhaite obtenir des polycondensats multiséquencés dont la chaîne comporte également la répétition d'une séquence polysiloxane, on fait réagir dans cette même étape un polymère polysiloxane présentant une fonction hydroxy ou amine aux extrémités de sa chaîne (i.e : un α,ω-dihydroxypolysiloxane, un α,ω-diaminopolysiloxane ou un alcoolaminepolysiloxane) avec le diisocyanate en quantité suffisante pour réagir avec les deux types d'oligomères polysiloxanes réactifs.

On obtient ainsi un polycondensat multiséquencé comportant en plus dans la chaîne la répétition d'une séquence polysiloxane de formule :

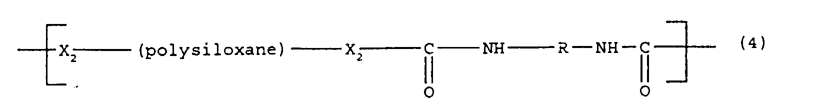

Si l'on souhaite obtenir des polycondensats multiséquencés dont la chaîne est étendue par la répétition d'une séquence -[-N-]- polyuréthane et/ou polyurée comportant des groupes non-ioniques et/ou des groupes ioniques et/ou d'une séquence -[-G-]- de polyuréthane et/ou polyurée comportant des oligomères de polymère organique, on couple les chaînes du polycondensat obtenu précédemment au moyen d'un agent coupleur (en quantité variable choisie en fonction de la longueur de la chaîne finale désirée) choisi parmi les diols et/ou les diamines et/ou les alcanolamines, de manière à obtenir finalement un nouveau polycondensat de plus longue chaîne comportant des groupes ioniques et/ou des groupes non-ioniques et/ou des oligomères de polymère organique.

Dans la deuxième étape, les fonctions alcools et/ou amines de l'agent coupleur (agent coupleur que l'on peut ici symboliser de manière commode par OH-B-OH, NH₂-B-NH₂,NH₂-B-OH , OH-E-OH, NH₂-E-NH₂, NH₂-E-OH) viennent alors réagir, et ceci selon les mêmes mécanismes que ceux exposés pour la première étape, soit avec les fonctions isocyanates portées en bout de chaîne par le polycondensat greffé siliconé obtenu ci-dessus, soit avec des fonctions isocyanates portées par du diisocyanate libre, lorsque ce dernier a été introduit en excès stoechiométrique lors de la première étape, donnant ainsi naissance dans la chaîne (plus longue) du nouveau polycondensat obtenu à une succession de motifs uréthane et/ou urée, c'est-à-dire à des séquences de type polyuréthane et/ou polyurée symbolisables par les formules : 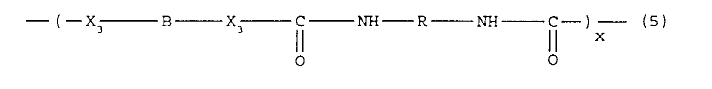 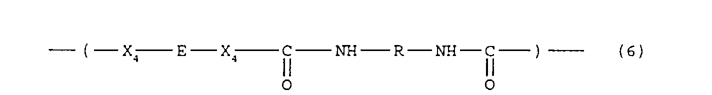 dans lesquelles X₃ et X₄ représentent -O- ou -NH-, et x est une valeur correspondant substantiellement au nombre de moles d'agent coupleur mis en oeuvre dans la réaction.

Comme indiqué précédemment, on obtient donc ainsi finalement un polycondensat multiséquencé constitué par la répétition de séquences greffées siliconées de formule (3), éventuellement de séquences polysiloxane (4) et/ou de séquences polyuréthane et/ou polyurées de formule (5) et/ou de formule (6).

Les agents coupleurs correspondant à la formule (5) (c'est-à-dire en fait le radical B) peuvent comporter des groupements chimiquement anionisables ou cationisables, c'est-à-dire des groupements qui, et respectivement, lorsqu'ils sont soumis à l'action d'une base, donnent des groupements anioniques (c'est le cas par exemple des groupements carboxyliques) et lorsqu'ils sont soumis à l'action d'un acide, donnent des groupements cationiques (cas par exemple d'une amine tertiaire). La neutralisation des groupements anionisables (respectivement cationisables) par la base (respectivement par l'acide) peut alors être, au choix, soit partielle soit totale, selon les quantités d'agents neutralisant mis en oeuvre.

Le caractère ionisable (et ionisé après neutralisation) du polycondensat permet ainsi de s'affranchir de l'emploi d'agents tensio-actifs lors de la préparation des pseudo-latex correspondants (autodispersibilité). Ces pseudo-latex sont obtenus selon les méthodes classiques et connues de préparation des pseudo-latex, sous réserve toutefois de certaines particularités qui seront mentionnées plus en détails par la suite. En particulier, on peut souligner à nouveau que les pseudo-latex conformes à l'invention présentent un caractère ionique plus ou moins marqué.

Les autres agents coupleurs correspondant à la formule (6) (c'est-à-dire en fait le radical E) sont des oligomères d'un polymère organique comportant une fonction diol, diamine ou alcoolamine en extrémité de chaîne.

De préférence, la séquence polyuréthane et/ou polyurée -[-M-]- comportant un greffon polysiloxane répond à la formule générale (I) suivante : 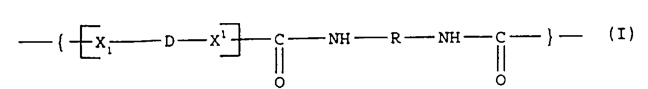 dans laquelle :
X₁ représente, séparément ou conjointement, -O- ou -NH- :
D est un segment de formule : 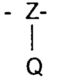
Z est un radical trivalent hydrocarboné pouvant contenir un ou plusieurs hétéroatomes tels que l'oxygène, le souffre, l'azote;
Q est un segment polysiloxane ;
R (qui n'est autre que le motif du diisocyanate tel que mentionné ci-avant) est un radical divalent choisi parmi les radicaux alkylènes de type aliphatique, cycloaliphatique ou aromatique.

De préférence, le segment polysiloxane Q répond à la formule générale (I') suivante : 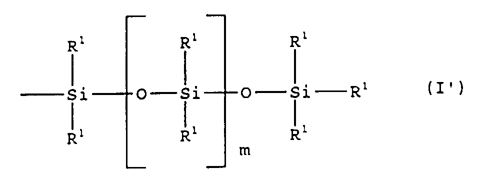 où R¹, identiques ou différents sont choisis parmi les radicaux hydrocarbonés, hydrohalogénocarbonés ou perhalogénés monovalents en C₁-C₂₀, exempts ou substantiellement exempts d'insaturations éthyléniques, et d'autre part les radicaux aromatiques, et m est un nombre entier tel que le poids moléculaire moyen mesuré en sommet de pic G.P.C du segment polysiloxane soit compris entre 300 et 50.000, de préférence entre 500 et 20.000.

De préférence, Z est un radical trivalent choisis parmi les radicaux alkyle ou alcoxy du type : 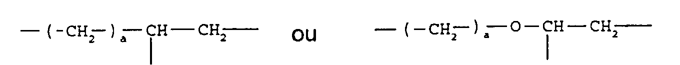 où a représente un nombre entier allant de 1 à 10.

A titre de radicaux R¹ convenant dans le cadre de l'invention, on peut plus particulièrement citer les radicaux alkyle, et notamment les radicaux méthyle, éthyle, propyle, isopropyle, butyle, pentyle, hexyle, octyle, décyle, dodécyle et octadécyle, les radicaux cycloalkyle, en particulier le radical cyclohexyle, les radicaux aryle, notamment phényle et naphtyle, les radicaux arylalkyle, notamment benzyle et phényléthyle, ainsi que les radicaux tolyle et xylyle. On notera que, selon l'invention, il est important que le segment polysiloxane soit exempt, ou substantiellement exempt, de motifs du type Si-H ou Si-R¹ dans lequel R¹ représenterait un radical présentant des insaturations éthyléniques, et ceci de manière à éviter toute réticulation intempestive du polycondensat sur lui-même.

Concernant maintenant les séquences polysiloxanes -[-L-]- rentrant dans la constitution des polycondensats qui sont utilisés dans le cadre de l'invention, celles-ci répondent, de préférence à la formule générale (II) suivante : 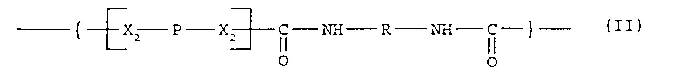 dans laquelle :
- P est un segment polysiloxane ;
- X₂ représente, séparement ou conjointement, -O- ou -NH- ;
- et R (qui n'est autre que le motif du diisocyanate tel que mentionné ci-avant) est un radical divalent choisi parmi les radicaux alkylènes de type aromatique, aliphatique ou cycloaliphatique.

De préférence, le segment polysiloxane P répond à la formule générale (II') suivante : 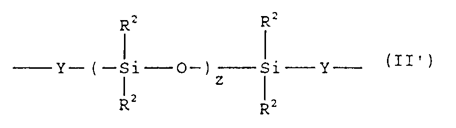 dans laquelle les radicaux R², qui peuvent être identiques ou différents, ont la même signification que celle de R¹ définie ci-dessus, Y représente un radical hydrocarboné divalent pouvant contenir un ou plusieurs hétéroatomes tels que l'oxygène, le souffre ou l'azote et z un nombre entier tel que le poids moléculaire moyen mesuré en sommet de pic G.P.C. du segment polysiloxane soit compris entre 300 et 50.000, de préférence entre 500 et 20.000.

De préférence, Y est un radical divalent choisi parmi les radicaux alkylènes de formule -(CH₂)_{b}-, dans laquelle b représente un nombre entier pouvant être compris entre 1 et 10.

Selon un mode particulièrement préféré de l'invention le segment polysiloxane P présent dans les polycondensats répond à la formule (II') suivante : 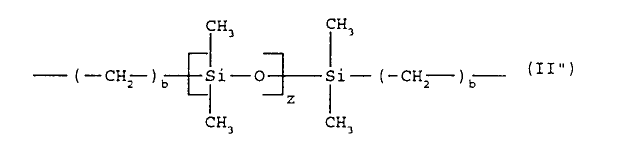 dans laquelle b et z sont des valeurs telles que définies ci-avant.

Concernant les séquence polyuréthanes et/ou polymères -[-N-]- pouvant rentrer dans la constitution des polycondensats de l'invention, celles-ci répondent de préférence à la formule générale (III) suivante : 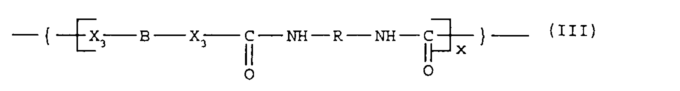 dans laquelle :
- X₃ représente, séparément ou conjointement, -O- ou -NH- ;
- R (qui, comme ci-avant pour la formule (I), n'est autre que le motif du diisocyanate utilisé pour conduire la réaction de condensation) est tel que défini ci-dessus pour les séquences de formule (I) ;
- x (qui, comme indiqué précédemment dans la description correspond substantiellement au nombre de moles d'agents coupleurs utilisées dans le procédé de synthèse du polycondensat) est un nombre entier pouvant varier de 1 à 10, et de préférence de 1 à 4,
- et B (qui n'est autre que le motif apporté par l'agent coupleur tel que mentionné ci-avant) est un radical hydrocarboné divalent porteur d'une charge ionique, positive ou négative, ou bien un radical hydrocarboné divalent non-ionique.

A titre de radicaux B porteurs de groupements anioniques (i.e de charges négatives), on peut plus particulièrement citer ceux qui sont porteurs de groupement présentant une ou des fonction(s) carboxylique(s) et/ou une ou des fonction(s) sulfonique(s), lesdites fonctions carboxyliques et/ou sulfoniques étant neutralisées partiellement ou totalement par une base minérale ou organique, comme cela sera expliqué plus en détails par la suite.

Ainsi, parmi les radicaux B divalents porteurs de fonctions carboxyliques ou sulfoniques convenant particulièrement bien dans le cadre de la présente invention, on peut mentionner ceux de formule (IV) : 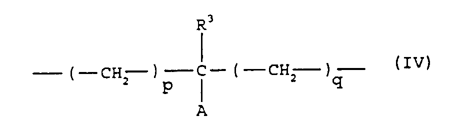 dans laquelle R³ représente un radical alkyle, linéaire ou ramifié, en C₁-C₃, A une fonction acide carboxylique (-COOH) ou acide sulfonique (-SO₃H) ou un sel desdites fonctions acides (fonctions carboxylate et sulfonate, respectivement), et p et q, qui peuvent être identiques ou différents, des nombres entiers compris entre 1 et 5,
et ceux de formule (IV') : 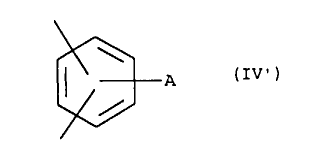 dans laquelle A a la signification ci-dessus.

A titre de radicaux B porteurs de groupements cationiques (i.e de charges positives), on peut plus particulièrement citer ceux qui sont porteurs de groupements de type amines tertiaires, lesdites amines tertiaires étant, pour partie ou totalement, soit neutralisées (présence de motifs -NH⁺-) soit quaternisées, soit bétaïnisées, comme cela sera expliqué plus en détails par la suite.

Ainsi, parmi les radicaux B divalents porteurs de fonctions amines tertiaires cationisables convenant particulièrement bien dans le cadre de la présente invention, on peut mentionner ceux de formule : 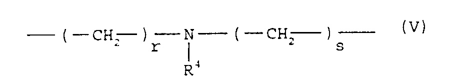 dans laquelle R⁴ représente un radical alkyle, linéaire ou ramifié, en C₁-C₄, et r et s deux nombres entiers, identiques ou différents, qui peuvent être compris entre 1 et 10.

Sous forme neutralisée , quaternisée ou bétaïnisée, les radicaux B ci-dessus deviennent alors : 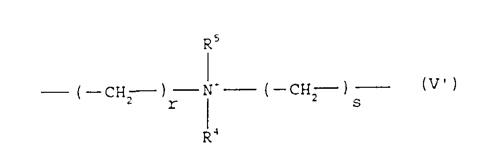 formule dans laquelle R⁴ a la signification ci-dessus, et R⁵ représente soit l'hydrogène (neutralisation) soit un radical alkyle, linéaire ou ramifié, en C₁-C₁₀ ou un cycle aromatique (quaternisation) ; soit un radical alkyle, linéaire ou ramifié, en C₁-C₁₀, porteur d'un groupe carboxylate ou sulfonate (bétaïnisation).

A titre de radicaux B non-ioniques, on peut citer les motifs apportés par les α, ω-diols aliphatiques, cycloaliphatiques ou aromatiques tels que l'éthylèneglycol, le propylèneglycol, le 1,4-butanediol, le cyclohexanediméthanol ; les α,ω-diamines aliphatiques, cycloaliphatiques ou aromatiques telles que le diaminopropane ; les alcoolamines aliphatiques, cycloaliphatiques ou aromatiques telles que les alcanolamines (éthanolamine).

Dans les définitions ci-dessus de B, lorsque la séquence -[-N-]- de formule (III) est auto-répétitive (x supérieur à 1), plusieurs types de B différents peuvent être présents dans la structure finale du polycondensat, avec en particulier une association entre des radicaux B non-ioniques et d'autres radicaux B porteurs de groupes anioniques ou cationiques. Il est préférable d'éviter dans la même chaîne la coexistence de groupes B anioniques et de groupes B cationiques.

Les séquences -[-N-]- présentes dans la structure de la chaîne des polycondensats multiséquencés de l'invention sont des séquences polyuréthanes et/ou polyurées apportant la rigidité, la polarité et éventuellement le caractère polyélectrolyte pour la possibilité de mise en dispersion dans l'eau.

Concernant les séquences polyuréthanes et/ou polyurées comportant des oligomères de polymère organique -[-G-]- pouvant rentrer dans la constitution des polycondensats multiséquencés de l'invention, celles-ci répondent, de préférence, à la formule générale (VI) suivante : 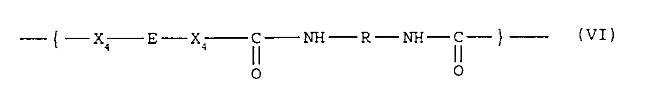 dans laquelle :
- X₄ représente, séparemment ou conjointement, -O- ou -NH-,
- R (qui, comme ci-avant pour la formule (I) n'est autre que le motif du diisocyanate utilisé pour conduire la réaction de condensation) est tel que défini précédemment,
- et E (qui n'est autre que le motif apporté par l'agent coupleur oligomère tel que mentionné ci-dessus) est un oligomère d'un polyéther, d'un polyester aliphatique et/ou cycloaliphatique et/ou aromatique obtenu par condensation d'un ou plusieurs diacides aliphatiques, cycloaliphatiques et/ou aromatiques et d'un ou plusieurs diols aliphatiques, cycloaliphatiques et/ou aromatiques ; un polyesteramide ou un polyamide, de préférence hydrophile.

Parmi les polyéthers, on peut citer, par exemples, les polyoxyéthylènes, les polyoxypropylènes, les copolymères oxyde d'éthylène/oxyde de propylène, les polyoxytétraméthylène. Parmi les polyesters, on peut citer le poly ε-caprolactame.

Les séquences -[-G-]- de formule (VI) étant répétitives, des valeurs différentes de E peuvent intervenir dans la structure du polymère final.

Les longueurs de chaîne de chaque oligomère E sont telles que leur poids moléculaire moyen mesuré en sommet de pic G.P.C. soit compris,de préférence, varie de 300 à 70.000.

Les segments oligomères E peuvent contenir des greffons ionisables sur la chaîne ou des groupes ionisables dans la chaîne favorisant la mise en dispersion dans l'eau du polymère final.

Les séquences -[-G-]- présentes dans la structure de la chaîne des polycondensats multiséquencés de l'invention sont également des séquences polyuréthanes et/ou polyurées apportant la rigidité, la polarité et éventuellement le caractère polyélectrolyte pour la possibilité de mise en dispersion dans l'eau.

Concernant enfin les radicaux R plus particulièrement préférés selon la présente invention et rentrant dans le cadre de la définition des séquences de formules (I) à (VI) données ci-avant, on peut mentionner ceux de formules : 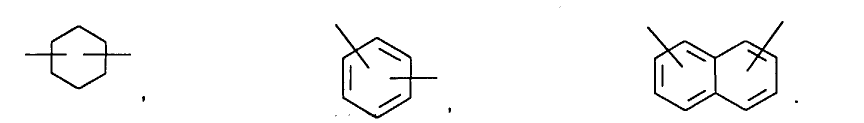 ou 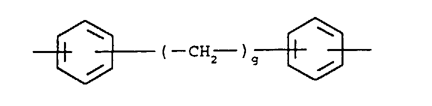 ou 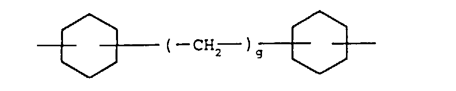 ou

-(-CH₂-)ₕ-

dans lesquelles g est un nombre entier compris entre 0 et 3, et h un nombre entier compris entre 1 et 20, de préférence compris entre 2 et 12.

Parmi les radicaux divalents R particulièrement préférés rentrant dans le cadre des formules ci-dessus, on peut citer les radicaux hexaméthylène, 4,4'-biphénylèneméthane, 2,4- et/ou 2,6-tolylène, 1,5-naphtylène, p-phénylène, méthylène 4,4-biscyclohéxyle et le radical divalent dérivé de l'isophorone.

Les polycondensats séquencés selon l'invention préférentiels sont caractérisés par le fait que leur chaîne est constituée par la répétition d'au moins une séquence -[-M-]- porteuse d'un greffon siliconé et d'au moins une séquence polyuréthane et/ou polyurée -[-N-]-et/ou d'une séquence polyuréthane et/ou polyurée -[-G-]- apportant les propriétés de rigidité et pouvant permettre la mise en dispersion dans l'eau (pseudo-latex).

Parmi les polycondensats selon l'invention du type polyuréthane et/ou polyurée à greffons polysiloxanes tels que définis précédemment, un certain nombre sont nouveaux et constituent un objet de l'invention. Il s'agit des polycondensats polyuréthane et/ou polyurée à greffons polysiloxanes, caractérisés par le fait que la chaîne est constituée par la répétition d'au moins une séquence polyuréthane et/ou polyurée -[-M-]- comportant un greffon polysiloxane et également :
(i) par la répétition d'une séquence de polyuréthane et/ou polyurée -[-N-]-répondant à la formule (III) générale suivante : 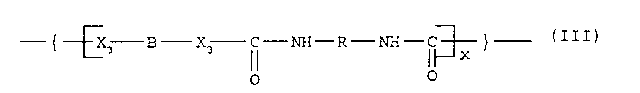 dans laquelle :
   X₃ représente, séparément ou conjointement, -O- ou -NH- ;
   R est un radical divalent choisi parmi les radicaux alkylènes de type aliphatique, cycloaliphatique ou aromatique ;
   x est un nombre entier allant de 1 à 10 ;
   les radicaux B, identiques ou différents, sont des radicaux divalents hydrocarbonés porteurs de groupes non-ioniques choisis parmi les motifs apportés par les α, ω-diols; les α, ω-diamines et les alcoolamines aliphatiques, cycloaliphatiques ou aromatiques ; porteurs de groupes anioniques présentant une ou des fonction(s) carboxylique(s) et/ou une des fonctions sulfoniques, lesdites fonctions carboxyliques et/ou sulfoniques étant neutralisées partiellement ou totalement par une base minérale ou organique ou bien porteurs de groupements amines tertiaires, lesdites amines tertiaires étant, partiellement ou totalement, soit neutralisées par une base minérale ou organique, soit quaternisées, soit bétaïnisées ; et/ou
(ii) par la répétition d'une séquence de polyuréthane et/ou polyurée -[-G-]- comportant des oligomères de polymères organiques ; et
(iii) par la répétition d'une séquence polysiloxane -[-L-]-.

Un procédé de synthèse des polycondensats séquencés utilisés dans le cadre de l'invention va être maintenant développé plus en détail. Dans ces grandes lignes, ce procédé correspond à celui déjà indiqué en début de description.

On fait réagir dans un solvant organique, un oligomère polysiloxane ayant une fonction diol terminale et/ou un oligomère polysiloxane ayant une fonction diamine terminale de formule générale : 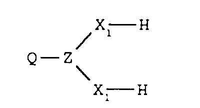 dans laquelle Q, Z et X₁ ont les significations définies ci-dessus,
avec un diisocyanate de formule :

O=C=N-R-N=C=O

dans laquelle R a la signification donnée ci-avant.

Dans le cas où l'on désire un polycondensat final comportant des séquences polysiloxanes -[-L-]-, on fait réagir simultanément avec le diisocyanate en quantité suffisante, un α, ω-dihydroxy et/ou diamino et/ou aminohydroxypolysiloxane de formule :

H-X₂-P-X₂-H

dans laquelle X₂ et P ont les significations indiqués ci-dessus, sous agitation à une température allant de 40 à 100° C en présence éventuellement d'un catalyseur du type sel d'étain.

Pendant cette réaction, le diisocyanate peut être utilisé pur ou de préférence en solution à 50 % dans le même solvant organique de synthèse.

Si le diisocyanate est aromatique (très réactif), on n'utilise pas de catalyseur. Si celui-ci est aliphatique ou cycloaliphatique, on utilise de préférence comme catalyseur un sel d'étain tel que le dibutyl dilaurate d'étain ou l'éthyl-2-hexanoate d'étain.

Dans le cas où l'on désire obtenir un polycondensat final comportant des séquences polyuréthanes et/ou polyurées -[-N-]- et/ou -[-G-]-, on couple dans une deuxième étape les chaînes du polycondensat obtenu précédemment par un diol et/ou une diamine et/ou une alcoolamine ou des mélanges de ces réactifs de formule :

H-X₃-B-X₃-H

H-X₄-E-X₄-H

dans lesquelles B, E, X₃ et X₄ ont les mêmes significations indiquées ci-dessus.

Les coupleurs sont introduits dans le milieu réactionnel en solution de préférence concentrée (de préférence supérieure ou égale à 50 % en poids) dans un solvant qui est soit identique à celui de la première étape soit compatible avec la solution initiale. On laisse réagir à nouveau pendant au moins deux heures à une température de 40 à 100° C.

Lorsque la première étape est réalisée, on introduit la quantité requise de diisocyanate pour l'extension des chaînes ou bien cette quantité est déjà présente au départ.

Pour l'obtention de séquences -[-N-]-, on utilisera de préférence des coupleurs H-X₃-B-X₃-H sous forme de mélanges de coupleurs non-ioniques et de coupleurs ionisables.

Si l'on veut au final, un polymère anionique, on utilise avantageusement l'acide diméthylol-propionique comme coupleur anionisable de formule :

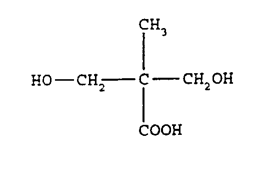

Si l'on veut au final, un polymère cationique, on utilise avantageusement la N-méthyldiéthanolamine de formule :

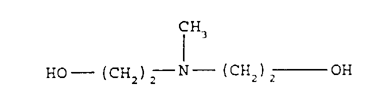

Une fois le coupleur ou le mélange de coupleurs introduit, on laisse réagir environ une heure sous agitation et à une température de préférence égale au point d'ebullition du solvant utilisé.

On ajoute alors un catalyseur pour obtenir une réaction complète de couplage des chaînes. Ce catalyseur est de préférence un sel d'étain tel que défini ci-dessus.

On laisse réagir à nouveau environ huit heures sous agitation à une température allant de 40 à 100° C.

Le solvant organique utilisé à ces étapes est de préférence choisi dans le groupe constitué par l'acétone, la méthyléthylcétone, le tétrahydrofuranne et le 1,2-dichloroéthane, ces solvants étant inertes vis-à-vis des groupes isocyanates.

Dans le cadre de la mise en oeuvre du procédé ci-dessus, les diisocyanates particulièrement préférés sont choisis, seuls ou en mélanges, parmi le diphénylméthane 4,4'-diisocyanate et le méthylène 4,4'-bis-dicydohexyldiisocyanate, et les agents coupleurs particulièrement préférés sont choisis, seuls ou en mélanges, parmi l'acide diméthylol propionique, la N-méthyldiéthanolamine, le 1,3-diaminopropane et l'éthanolamine, étant bien entendu que la possibilité de mélange coupleur acide/coupleur amine est déconseillée.

Pour que la réaction de la deuxième étape soit totale, on doit rester en milieu homogène. On peut dans cet objectif rajouter un autre solvant en complément pouvant être, par exemple le diméthylformamide ou la N-méthylpyrrolidone en quantité suffisamment faible pour homogénéiser le milieu réactionnel.

Lorsque le polymère final obtenu est utilisé sous forme de solution dans un solvant organique, on purifie ledit polymère formé par précipitation dans un non-solvant et séchage du précipité ainsi obtenu. Le polymère final purifié peut être ensuite dissous dans le ou les solvants choisis pour l'application cosmétique.

Lorsque le polycondensat final obtenu est utilisé sous forme d'une dispersion aqueuse (pseudo-latex), il peut être purifié par exemple dans un solvant non-polaire tel que le cyclohexane puis préparé selon le procédé qui sera développé ci-dessous.

On entend désigner selon l'invention, et ceci en conformité avec l'acception commune, par l'expression "pseudo-latex" une suspension aqueuse stable constituée de fines particules, généralement sphériques, du polycondensat tel que ci-dessus défini, ces particules ayant été obtenues par mise en dispersion, dans une phase aqueuse appropriée, dudit polycondensat à l'état déjà synthétisé.

L'expression "pseudo-latex" ne doit donc pas être confondue avec l'expression "latex" ou "latex synthétique" qui est certes également une suspension aqueuse constituée de particules d'un polymère ou d'un polycondensat, mais dont lesdites particules ont été classiquement obtenues directement par polymérisation (respectivement par polycondensation) en émulsion d'un ou plusieurs monomères dans une phase aqueuse appropriée.

En particulier, la synthèse d'un "latex" nécessite obligatoirement l'emploi d'agents tensio-actifs, lesquels se retrouvent alors toujours dans la suspension finale.

Les polycondensats de l'invention appropriés pour la mise en dispersion dans l'eau sont ceux dont la chaîne est constituée de séquences -[-N-]- et/ou -[-G-]- de polyuréthanes et/ou polyurées comportant suffisamment de groupes ionisables.

Conformément à l'invention, ces polycondensats, éventuellement purifiés, peuvent être ensuite utilisés à la préparation d'un pseudo-latex stable qui sera constitué de particules solides dudit polycondensat neutralisé à l'aide d'un agent neutralisant convenable qui peut être soit une base minéral ou organique lorsque le radical B (et/ou le radical E) tel que ci-avant défini est porteur de fonctions anionisables telles que par exemple des fonctions acides carboxyliques et/ou sulfoniques, soit un acide minéral ou organique lorsque ledit radical B (et/ou le radical E) est porteur de fonctions cationisables telles que par exemple des fonctions amines tertiaires, soit un halogénure d'alkyle en vue de procéder spécifiquement à la quaternisation d'amines tertiaires. Selon l'invention, le taux de neutralisation peut aller de 10 % à 100 %, de préférence de 20 à 100 %.

A l'inverse d'un procédé conventionnel de préparation de pseudo-latex qui consiste à dissoudre un polymère insoluble dans l'eau, dans un solvant organique, soluble ou partiellement soluble dans l'eau, à introduire dans la solution organique de polymère ainsi obtenue un tensio-actif, un mélange de tensioactifs ou un polymère colloïde protecteur ou bien encore un mélange tensio-actif(s)/polymère colloïde protecteur, et ceci dans le but d'obtenir une bonne stabilisation des particules, puis à disperser (émulsion) sous agitation la dispersion ainsi obtenue dans de l'eau et à procéder ensuite à l'élimintion du solvant organique par évaporation sous vide, ce qui conduit à une suspension aqueuse de particules de polymère enrobées de tensio-actif(s) et/ou de polymère colloïde protecteur, les polycondensats à séquences Polyuréthane/Polyurée utilisées dans le cadre de l'invention, puisqu'ils comportent des fonctions ioniques partiellement ou totalement neutralisées apportant aux polycondensats une sorte d"'autodispersabilité" dans l'eau, permettent d'obtenir des pseudo-latex particulièrement stables en l'absence de tout stabilisant hydrophile, de tensio-actif ou de colloïde protecteur.

Il va de soi que la nature de l'agent neutralisant qu'il conviendra d'utiliser pour neutraliser le polycondensat Polyuréthane/Polyurée sera fonction de la nature des fonctions ionisables portées par ce dernier.

Lorsque ledit polycondensat comporte une fonction anionisable telle que par exemple une fonction acide carboxylique ou sulfonique, l'agent neutralisant peut être une base minérale telle que la soude, la potasse ou l'ammoniaque, ou une base organique telle qu'un aminoalcool choisi notamment parmi le 2-amino 2-méthyl 1-propanol (AMP), la triéthanolamine, la triisopropanolamine (TIPA), la monoéthanolamine, la diéthanolamine, la tri[(2-hydroxy) 1-propylamine, le 2-amino 2-méthyl 1,3-propanediol (AMPD), et le 2-amino 2-hydroxyméthyl 1,3-propanediol ou bien une diamine telle que la lysine.

Lorsque le polycondensat comporte une fonction cationisable du type amine tertiaire, l'agent neutralisant peut être un acide minéral tel que l'acide chlorhydrique, ou un acide organique tel que l'acide lactique, l'acide glycolique ou l'acide mandélique. L'agent neutralisant peut être aussi un agent quaternisant de la fonction amine tertiaire, comme par exemple les halogénures d'alkyles et en particulier le iodure de méthyle ou le bromure d'éthyle.

La neutralisation peut être réalisée soit in-situ dans la solution du polycondensat Polyuréthane/Polyurée dans le solvant organique par addition de la quantité déterminée d'agent neutralisant, soit lors de la préparation de l'émulsion, l'agent neutralisant se trouvant alors dans la phase aqueuse de l'émulsion.

Le solvant organique utilisé doit être un solvant volatil ou un mélange de tels solvants présentant un point d'ébullition inférieur à celui de l'eau et être par ailleurs miscible ou partiellement miscible à l'eau. Un tel solvant organique est de préférence choisi parmi l'acétone, la méthyléthylcétone, le tétrahydrofuranne, l'acétate de méthyle, l'acétate d'éthyle, l'isopropanol et l'éthanol.

Après l'obtention du polycondensat Polyuréthane/Polyurée greffé siliconé partiellement neutralisé dans le solvant organique, on procède alors à la préparation d'une émulsion en versant, sous agitation, à la solution organique obtenue, une quantité appropriée d'eau contenant éventuellement un agent anti-mousse dont le rôle sera de faciliter l'évaporation ultérieure de la phase organique.

Comme indiqué précédemment, on peut, selon une variante du procédé, opérer la neutralisation des fonctions ionisables du polycondensat lors de la formation même de l'émulsion en versant une solution aqueuse contenant la quantité requise de l'agent neutralisant.

Lors de la formation de l'émulsion, l'agitation est de préférence réalisée à l'aide d'un disperseur cisaillant du type Moritz ou Ultra Turrax ou Raineri équipé de pâles défloculantes.

L'émulsion ainsi obtenue est particulièrement stable sans qu'il soit nécessaire d'employer un agent tensio-actif dans la mesure où les groupes ioniques du polycondensat Polyuréthane/Polyurée se placent à l'interface avec l'eau et protègent les gouttelettes de la coalescence par répulsion électrostatique.

Après formation de l'émulsion à une température comprise entre la température ambiante et 70° C environ, on procède alors à l'évaporation sous pression réduite du solvant organique jusqu'à son élimination totale, ladite évaporation étant de préférence réalisée sous léger chauffage.

On obtient ainsi finalement un pseudo-latex, c'est-à-dire une dispersion aqueuse de particules du polycondensat Polyuréthane/Polyurée greffé siliconé filmogène, qui est exempte de tout tensio-actif ou de tout autre stabilisant hydrophile, tout en étant très stable.

La taille moyenne des particules constituant le pseudo-latex, ainsi que leur polydispersité, peuvent être réglées en faisant varier, lors de la préparation dudit pseudo-latex, les proportions respectives entre le polycondensat, le solvant organique et l'eau, ou en faisant varier le taux de neutralisation ou la nature de l'agent neutralisant.

Les pseudo-latex ainsi obtenus par le procédé de préparation décrit ci-dessus ainsi que ledit procédé constituent d'autres objets de l'invention.

Selon un mode particulier de réalisation des pseudo-latex utilisés dans le cadre de la présente invention, la taille moyenne des particules constituant ledit pseudo-latex est comprise entre 5 et 400 nanomètre, de préférence entre 10 et 250 nanomètres.

La polydispersité en taille desdites particules, mesurée par diffusion quasi-élastique de la lumière, est, quant à elle, généralement inférieure à 0,5, et de préférence inférieure à 0,3.

L'objet principal de l'invention consiste en l'utilisation des polycondensats séquencés à greffons siliconés ou leurs formes pseudo-latex tels que décrits précédemment dans et pour la fabrication de compositions cosmétiques ou dermatologiques.

Les compositions cosmétiques ou dermatologiques selon l'invention peuvent donc se présenter sous forme de dispersion aqueuse et contenir les polymères de l'invention sous forme de pseudo-latex.

Ce type de composition est particulièrement adaptée aux polycondensats multiséquencés selon l'invention comportant dans leur chaîne des séquences polyuréthanes et/ou polyurées à groupements ionisables.

Les compositions cosmétiques ou dermatologiques de l'invention peuvent se présenter sous forme de solution du polycondensat greffé siliconé dans un solvant organique ; sous forme d'«émulsion» ou de dispersion dans l'eau d'une solution du polycondensat greffé siliconé dans un solvant organique ou bien sous forme de solution hydroorganique du polycondensat greffé siliconé.

On utilisera de préférence des solvants organiques des polycondensats greffés siliconés de l'invention, cosmétiquement ou dermatologiquement acceptables comme ceux du type éther.

Suivant le type d'application choisi, on peut utiliser un solvant miscible à l'eau ou bien un mélange de solvants miscibles à l'eau dont l'un (servant de diluant) s'évapore avant l'eau de façon à permettre au polymère d'être dissous dans un solvant en présence d'eau pendant toute la durée de séchage de la formulation appliquée sur la matière kératinique traitée.

Parmi les solvants miscibles à l'eau, on peut citer le diméthoxyéthane et un mélange de diméthoxyéthane/diéthoxyéthane.

On peut également choisir un solvant ou mélange de solvants des polymères selon l'invention, non miscibles à l'eau tels que le diéthoxyéthane. Si la formulation envisagée nécessite pour l'application donnée la présence d'eau, la solution organique du polymère peut être dispersée, émulsionnée dans l'eau avec des agents stabilisants tels que des agents tensio-actifs, des agents gélifiants présents dans la phase aqueuse. Dans ce cas, l'un des solvants organiques utilisés pour dissoudre le polymère présente préférentiellement, un point d'ébullition supérieur à celui de l'eau. On peut utiliser notamment le diéthoxyéthane.

Les compositions selon l'invention contiennent donc dans un support cosmétiquement acceptable les polymères tels que décrits ci-dessous, pour des applications aussi variées que celles rencontrées par exemple dans le domaine du capillaire, du maquillage ou bien encore des soins de la peau, ou de tout autre domaine cosmétique dans lequel l'utilisation d'une substance filmogène est désirable ou recherchée, des propriétés tout à fait remarquables, en particulier au niveau de leurs propriétés filmogènes et de brillance, de leur aptitude à conserver ces propriétés dans le temps face à l'action d'agents extérieurs (rémanence) et aussi de leur propriétés de douceur, de lubrification et de résistance à l'abrasion.

Parmi les applications préférentiellement visées par la présente invention, et les différents effets bénéfiques obtenus dans ces dernières, on peut plus particulièrement mentionner :
- le domaine des produits capillaires (lavage, soin ou beauté des cheveux), où les compositions selon l'invention, en particulier sous forme d'aérosols, de mousse, de shampooings, d'après-shampooings, de lotions ou de gels coiffants ou traitants, laques ou lotions de mise en forme ou de mise en plis ou encore de fixation, permettent d'apporter aux cheveux brillance, douceur, facilité de coiffage (phénomène d"'individualisation" des cheveux au moment du dépôt de la composition), meilleur toucher, ainsi que la rémanence (c'est-à-dire le maintien durable, même sous l'action d'agents extérieurs) de ces propriétés.
- le domaine des produits de maquillage, en particulier pour le maquillage des ongles et des cils, où les compositions selon l'invention, sous forme de vernis à ongle, de mascaras ou de eye-liners par exemple, permettent d'apporter, dans le cas du maquillage des cils, les mêmes avantages que ceux évoqués précédemment pour le traitement des cheveux, et, dans le cas des vernis à ongles (où les compositions peuvent être utilisées comme filmogène seul ou comme additif filmogène), brillance, meilleure mouillabilité de l'ongle, rémanence du film et de sa brillance aux lavages, meilleure résistance à l'abrasion (apport de glissant par lubrification des surfaces), meilleure rigidité.
- dans le domaine des produits de soin de la peau (crèmes, laits lotions, masques, sérums, produits solaires), où les compositions selon l'invention permettent plus particulièrement d'apporter brillance, meilleure mouillabilité et résistance aux lavages à l'eau (produits solaires).

La proportion en polymère filmogène dans les compositions cosmétiques est généralement comprise entre 0,5 et 50%, et de préférence entre 1 et 20% en poids total de la composition. Dans le cas des vernis à ongles, cette proportion est en général supérieure ou égale à 25 % en poids.

Les compositions peuvent en outre, et bien entendu, contenir divers adjuvants destinés à la rendre acceptable dans une application cosmétique particulière.

Les compositions selon l'invention peuvent contenir des filtres solaires UV-A ou UV-B ou à bande large et être utilisées ainsi comme produits anti-solaires.

Les compositions selon l'invention peuvent par ailleurs contenir des additifs cosmétiques conventionnels choisis parmi les corps gras, des solvants organiques, des agents épaississants, des adoucissants, des agents anti-mousse, des agents hydratants, des humectants, des agents traitants (agents anti-chute, anti-pelliculaire,...), des antiperspirants, des agents alcanisants, des colorants, des pigments, des parfums, des conservateurs et des agents propulseurs lorsque les compositions se présentent sous forme aérosol.

Elles peuvent contenir en plus des silicones traditionnellement utilisées en cosmétique et/ou des polymères organiques anioniques, non-ioniques ou amphotères compatibles avec des polyuréthanes ou des polyurées. La présence des greffons siliconés dans la structure des polycondensats de l'invention permet d'obtenir une bonne compatibilité de ces polymères avec les silicones et les polymères organiques additifs, qui en général sont difficilement compatibles entre eux en l'absence des polymères de l'invention.

Plus précisément, comme corps gras, on peut utiliser une huile ou une cire ou leurs mélanges, des acides gras, des alcools gras, des esters d'acides gras tels que les triglycérides d'acides gras en C₆-C₁₈, de la vaseline, de la paraffine, de la lanoline, de la lanoline hydrogénée ou acétylé.

Parmi les huiles, on peut citer les huiles minérales, animales, végétales ou les huiles de synthèse et notamment l'huile de vaselin, de paraffine, de ricin, de jojoba, de sésame, ainsi que les huiles et les gommes de silicones et les isoparaffines.

Parmi les cires animales, fossiles, végétales, minérales ou de synthèse, on peut notamment citer la cire d'abeille, de caroube, de Candellila, l'ozokérite, les cires microcristallines ainsi que les cires et les résines de silicone.

Parmi les agents épaississants, on peut citer :
- les celluloses modifiées telles que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 44001H" par la Société Amercol,
- la gomme de caroube, la gomme de guar, la gomme de guar quaternisée vendue sous la dénomination de "Jaguar C-13-S"" par la Société Meyhall, la gomme d'hydroxyproplguar, la gomme de xanthane,
- les acides polyacryliques réticulés tels que les "Carbopol" de la Société Goodrich,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères réticulés d'acrylamide et d'acrylate d'ammonium vendus sous les dénominations de "PAS 5161" ou "Bozepol C" par la Société Hoechst, les polymères réticulés d'acrylamide et d'acide 2-acrylamido 2-méthylpropane sulfonique partiellement ou totalement neutralisés, vendus sous la dénomination de "Sepigel 305" par la Société Seppic, les polymères réticulés d'acrylamide et de chlorure de méthacryloyoxyéthyltriméthylammonium vendus sous la dénomination de "Salcare SC95" par la Société Allied Colloïd, ou encore,
- les homopolymères réticulés de chlorure de méthacryloyoxyéthltriméthylammonium vendus sous la dénomination de "Salcare SC95" par la Société Allied Colloïd.

Les exemples qui suivent servent à illustrer l'invention sans toutefois présenter un caractère limitatif.

### EXEMPLES

Les synthèses conduisant aux polycondensats séquencés polyuréthanes et/ou polyurées greffés siliconés ont été réalisés à partir de l'oligomère polysiloxane à fonction diol terminale de formule générale : 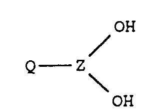 telle que définie précédemment, vendu par la Société SHINETSU sous la dénomination X22176 DX ayant un poids moléculaire moyen d'environ 4.000 et un indice d'OH de 26,7.

### Exemple 1:

Dans cet exemple, on a préparé un polycondensat de structure théorique : 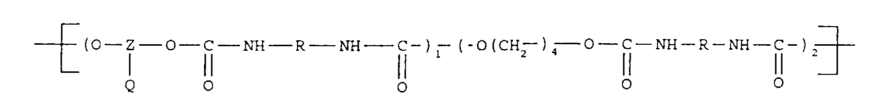 dans laquelle :
R représente : 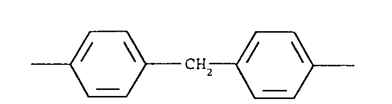 correspondant à la réaction entre :
- 1 mole de X 22176 DX (oligomère polysiloxane),
- 2 moles de 1,4-butane diol (agent coupleur),
- 1mole de 4,4'-diphénylméthane diisocyanate (ci-après dénommé MDI).

Dans un réacteur cylindrique, pourvu d'une agitation centrale du type ancre, d'un thermomètre, d'un réfrigérant, d'une arrivée de vide et d'une arrivée de barbotage d'azote et surmonté d'une ampoule à introduction, on introduit, sous courant d'azote, une solution de 100 g d'oligomère X 22176 DX dans 120 g de tetrahydrofuranne (nommé par la suite THF). On fait plusieurs dégazages vide/azote pour purger l'air à l'intérieur du réacteur. On agite à environ 250 tours/minutes. On introduit alors rapidement et sous courant d'azote, 17,9 g de MDI solide dans le milieu sous agitation à température ambiante.

On dissous totalement sous agitation et on chauffe à 65° C (ébullition du solvant) pendant 3 heures.

On ajoute alors rapidement (par l'ampoule à introduction) une solution de 4,82 g de 1,4-butanediol dans 50 g de THF. On laisse réagir sous agitation une heure à 65° C.

On ajoute alors 0,05 g de catalyseur (liquide) dibutylaurate d'étain pur et on laisse réagir à 65° C pendant huit heures. A ce stade, la réaction de couplage est terminée.

On ramène le milieu à température ambiante. La solution de synthèse est purifiée par précipitation dans un mélange éthanol/eau (70/30 en poids). On récupère le précipité et on sèche.

On obtient 105 g de polycondensat séquencé.

### Example 2 :

Dans cet exemple, on a procédé à la réalisation d'une émulsion contenant le polymère préparé à l'exemple 1.

15 g dudit polymère sont dissous dans du diéthoxyéthane (point d'ébullition 125° C) de façon à réaliser une solution à 4 % en poids de polymère. Cette solution est émulsionnée dans l'eau après stabilisation par un tensio-actif approprié pour constituer une émulsion finale à 2 % en poids de polymère.

Cettte émulsion peut être utilisée à l'application sur les cheveux après shampooing pour constituer une lotion de mise en forme de la coiffure.

### Exemple 3 :

Dans cet exemple, on a procédé à la préparation d'un polycondensat séquencé polyuréthane anionique à greffons siliconés de structure théorique 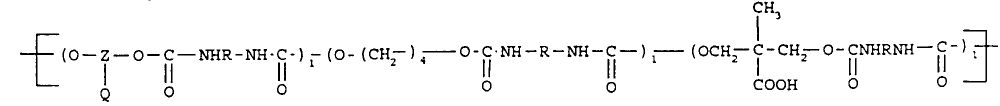 où R désigne : 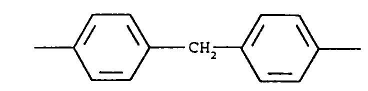 correspondant à la réaction entre :
- 1 mole de X 22176 DX,
- 1 mole de 1,4 butane diol (agent coupleur),
- 1 mole d'acide diméthylolpropionique (nommé par la suite DMPA ; agent coupleur),
- 3 moles de MDI.

On procède dans les mêmes conditions que celles de l'exemple 1 avec :
- 100 g de X 22176 DX,
- 120 g de THF,
- 17,9 g de MDI.

La réaction s'effectue à 65° C pendant 3 heures.

On introduit ensuite dans le milieu à 65° C sous agitation, une solution de mélange de coupleurs constituée par :
- 2,4 g de 1,4 butanediol,
- 3,6 g de DMPA,
- 200 g de THF.

On laisse réagir pendant 1 heure à 65°C.

Pour les exemples qui suivent, on réalise deux synthèses du polymère identiques.

### Exemple 4 :

Dans cet exemple, on réalise une émulsion d'un polymère préparé dans l'exemple 3. Une des deux synthèses de l'exemple 3 est purifiée par précipitation de la solution de synthèse dans l'eau. On obtient ainsi 115 g de polymères d'indice d'acide I_{A} = 10 après séchage. 100 g du polymère obtenu sont dissous dans un mélange 50/50 en poids de diméthoxyéthane (point d'ébullition = 85° C) et de diéthoxyéthane (point d'ébullition = 125° C) de façon à constituer une solution à 25 % en polymère. On neutralise le polymère dissous par l'amino-2 méthyl-2 propanol (appelé par la suite AMP) à un taux de neutralisation de 70 % d'après l'indice d'acide du polymère (soit 1,11 g d'AMP).

Cette solution est autoémulsionnée dans l'eau en procédant de la façon suivante. La solution organique est agitée vivement à l'aide d'un disperseur cisaillant du type Ultra Turrax, on ajoute les 1,11 g d'AMP dissous dans 50 ml d'eau en procédant par ajouts successifs, et on dilue ensuite par ajout de 500 g d'eau permutée, toujours en agitant fortement à l'Ultra Turrax. On obtient ainsi une émulsion à 12 % d'extrait sec. On peut la concentrer à un extrait sec de 25 % à l'évaporateur rotatif.

En incorporant dans ladite émulsion, des actifs non-ioniques hydrosolubles pour le soin de l'ongle, on obtient une formulation constituant une base de soin des ongles apportant un film protecteur.

De la même façon, cette émulsion, diluée par l'eau pour avoir un extrait sec final de 5 % en polymère, constitue une formule de mise en forme appliquée sur cheveux mouillés et séchés au sèche-cheveux (après application de l'émulsion).

### Exemple 5 :

Dans cet exemple, on réalise un pseudo-latex à partir du polymère obtenu dans la deuxième synthèse de l'exemple 3.

On introduit dans un bécher 450 g de la solution de synthèse (à une concentration de 24 % en polymère, l'indice d'acide du polymère lA = 10). On agite fortement cette solution par un agitateur dispersant du type Ultra Turrax. On introduit petit à petit une solution constituée par 0,85 g d'AMP dans 50 ml d'eau permutée (pour neutralisation à 50 % du polymère).

On dilue l'émulsion, toujours sous vive agitation à l'Ultra Turrax en ajoutant progressivement 900 g d'eau permutée.

L'émulsion ainsi obtenue est concentrée à l'évaporateur rotatif pour éliminer le solvant organique (THF) et une partie de l'eau. On obtient ainsi un pseudo-latex stable (350 g) d'extrait sec 37 % présentant les caractéristiques suivantes :
. tailles moyennes des particules 180 nm,
. polydispersité en taille < 0,1

Ces tailles de particules étant mesurées par diffusion quasi-élastique de lumière sur un COULTER N4 SD de la Société COUTRONIX.

### Exemple 6 :

Dans cet exemple, on a préparé un polycondensat de structure théorique : 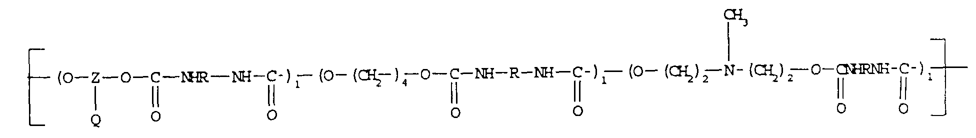 où R désigne : 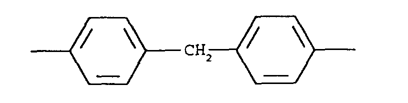 correspondant à la réaction entre :
- 1 mole de X 22176 DX,
- 1 mole de 1,4 butane diol (agent coupleur),
- 1 mole de N-méthyldiéthanolamine (nommé par la suite MER ; agent coupleur),
- 3 moles de MDI.

On procède dans les mêmes conditions que celles de l'exemple 1 avec :
- 100 g d'oligomère X 22176 DX,
- 120 g de THF,
- 17,9 g de MDI.

La réaction s'effectue à 65° C pendant 3 heures.

On introduit alors dans le milieu à 65° C sous agitation une solution de mélange de coupleurs constituée par :
. 2,145 g de 1,4 butanediol
. 2,84 g de N-méthyldiéthanolamine (MEA)
. 200 g de THF

On laisse réagir une heure à 65° C.

La suite étant identique au procédé de l'exemple 1 (pour le couplage).

On réalise deux synthèses de ce polymère.

### Exemple 7 :

Dans cet exemple, on réalise un pseudo-latex à partir de la première synthèse de l'exemple 6.

On introduit dans un bécher 455 g de la solution de synthèse précédente à 27 % en polymère (soit 123 g du polymère). On agite vivement cette solution à l'Utra Turrax. On introduit petit à petit, dans le milieu sous agitation, une solution constituée par :
. 11,6 g d'HCl 2M
. 50 ml d'eau permutée (pour neutraliser le polymère à 100 % d'après son taux d'amine).

On ajoute ensuite, toujours sous vive agitation 500 g d'eau permutée.

On concentre alors l'émulsion obtenue à l'évaporateur rotatif pour éliminer totalement le solvant organique de synthèse (THF) et pour concentrer en eau.

On obtient ainsi un pseudo-latex d'extrait sec final 27 % et présentant les caractéristiques suivantes.
. taille moyenne des particules : 100 nm,
. polydispersité en taille : 0,14

### Exemple 8 :

Dans cet exemple, on réalise une émulsion à partir de la deuxième synthèse de l'exemple 6.

On purifie le polymère par précipitation dans l'eau et on sèche.

On prend 100 g du polymère, on les dissout à une concentration de 25 % dans un mélange de diméthoxyéthane/diéthoxyéthane (50/50 en poids).

On agite la solution obtenue à l'Ultra Turrax.

On introduit petit à petit une solution constituée de :
. 11,6 g d'HCl 2M
. 50 ml d'eau permutée.
puis on la dilue progressivement par 500 g d'eau permutée de façon à obtenir une émulsion stable d'extrait sec 12 %.

Lorsque l'émulsion obtenue est diluée à 5 % par l'eau, on obtient une formulation de lotion de mise en forme pour cheveux.

### Exemple 9 :

On donne ici un exemple de formulation pour vernis à ongles.
- Pseudo-latex de l'exemple 5 25 g en matière active
- Epaississant associatif uréthane non ionique vendu sous la dénomination de "SER AD FX 1100" par la Société Servo 0,3 g
- Pigments 1g
- Eau qsp 100 g

Le vernis à ongles obtenu est très résistant à l'eau : le film est intact après 1 heure sous agitation dans l'eau.

Le film obtenu adhère correctement à la kératine de l'ongle sans s'écailler. Il n'est pas collant et résiste aux rayures.

Le vernis obtenu selon l'invention s'applique facilement sur l'ongle et présente en outre une très bonne brillance, ainsi qu'une tenue satisfaisante.

### Exemple 10 :

Cet exemple illustre une formulation pour mascaras.

### Phase A :

- stéarate de triéthanolamine 11,8 g
- cire d'abeille 5 g
- cire de carnauba 3 g
- paraffine 1 g

### Phase B :

- oxyde de fer noir 5 g

### Phase C :

- gomme arabique 2 g
- hydroxyéthylcellulose vendue sous la dénominationde "Cellosize QP" par la Société Amerchol 1,2 g

### Phase D :

- pseudo-latex de l'exemple 5 5 g de matière active
- conservateur qs
- eau qsp 100 g

Ce mascara est obtenu en portant les ingrédients de la Phase A à 85° C, à laquelle on ajoute la Phase B et l'on agite à l'aide d'une turbine.

On porte ensuite l'eau de préparation à ébullition, on ajoute les conservateurs, puis à 85° C les ingrédients de la Phase C.

On ajoute alors la phase aqueuse obtenue (85° C) à la Phase A (85° C) sous agitation à l'aide d'une turbine (émulsification) puis on ajoute enfin, à 30° C, le pseudo-latex de la Phase D et agite à l'aide d'une pâle.

### Exemple 11:

Cet exemple illustre une autre composition pour mascaras. On prépare ce mascara selon le même mode opératoire que celui donné à l'exemple 10, mais avec les constituants suivants :

### Phase A :

- stéarate de glycérol 3 g
- mélange d'esters d'acide laurique et de sorbitol et d'acide laurique et de sorbitol oxyéthyléné à 20 moles d'oxyde d'éthylène, vendu sous la dénomination "Tween 20" par la Société ICI 3,7 g
- monoesters d'acide stéarique et de sorbitane vendu sous la dénomination de "Span 60" par la Société ICI 5,6 g
- cire d'abeille 6 g
- cire de carnauba 1,8 g
- paraffine 7,8 g

### Phase B :

- Oxyde de fer noir 4,5 g

### Phase C :

- Hydroxyéthylcellulose vendue sous la dénominationde "Cellosize QP" par la Société Amerchol 1,5 g

### Phase D :

- pseudo-latex de l'exemple 7 20 g
- conservateurs qs
- eau qsp 100 g

### Exemple 12 :

On donne ici divers exemples de formulations capillaires.

### Shampooing :

- Pseudo-latex de l'exemple 5 5 g de matière active
- Lauryl éther sulfate de sodium 15 g
- Cocoylbétaïne en solution aqueuse à 32 % vendu sous le nom "Chimexane HC" par la Société CHIMEX 3 g de matière active
- Parfums, conservateurs, qs
- Eau déminéralisée qsp 100 g

### Shampooing :

- pseudo-latex de l'exemple 7 5 g de matière active
- lauryléther sulfate de sodium 15 g
- cocoylbétaïne en solution aqueuse de 32 % 3 g de matière active
- parfums, conservateurs, qs
- eau déminéralisée qsp 100 g

### Lotion de mise en forme :

- pseudo-latex de l'exemple 5 5 g de matière active
- parfums, colorants, conservateurs qs
- eau déminéralisée qsp 100 g

Cette composition, appliquée sur les cheveux, après un shampooing, apporte un bon maintien de la coiffure et une très bonne brillance aux cheveux.

### Lotion de mise en forme :

- pseudo-latex de l'exemple 7 5 g de matière active
- parfums, colorants, conservateurs qs
- eau déminéralisée qsp 100 g

Cette composition, appliquée sur les cheveux, après un shampooing, apporte un bon maintien de la coiffure et une très bonne brillance aux cheveux.

### Spray de coiffage :

On prépare un spray de coiffage en flacon pompe en conditionnant, dans un récipient approprié, la composition suivante :
- pseudo-latex de l'exemple 5 3 g de matière active
- parfums, colorants, conservateurs qs
- eau déminéralisée qsp 100 g

Le récipient, une fois rempli, est ensuite équipé d'une pompe de pulvérisation.

Cette composition donne une bonne tenue de la coiffure et une très bonne brillance aux cheveux.

### Spray de coiffage :

### On prépare un spray de coiffage en mélangeant :

- pseudo-latex de l'exemple 7 3 g de matière active
- parfums, colorants, conservateurs qs
- eau déminéralisées qsp 100 g

La lotion obtenue étant ensuite conditionnée dans un pulvérisateur rechargeable en air comprimé.

Cette composition donne une bonne tenue de la coiffure et une très bonne brillance aux cheveux.

## Revendications

1. Utilisation dans et pour la fabrication d'une composition cosmétique ou dermatologique d'un polycondensat séquencé polyuréthane et/ou polyurée comportant une chaîne constituée par la répétition d'au moins une séquence polyuréthane et/ou polyurée -[-M-]- comportant un greffon polysiloxane.

2. Utilisation selon la revendication 1, caractérisée par le fait qu'il est multiséquensé et que la chaîne est également constituée par la répétition d'une séquence de polyuréthane et/ou polyurée -[-N-]- comportant des groupes non-ioniques et/ou des groupes ioniques et/ou d'une séquence de polyuréthane et/ou polyurée -[-G-]-comportant des oligomères de polymères organiques de poids moléculaire moyen mesuré en sommet de pic G.P.C., compris entre 300 et 70.000 et/ou d'une séquence polysiloxane -[-L-]-.

3. Utilisation selon l'une quelconque des revendications 1 et 2, caractérisée par le fait que la séquence -[-M-]- de polyuréthane et/ou de polyurée greffée par un polysiloxane répond à la formule générale suivante : 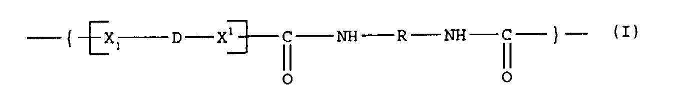 dans laquelle :
X₁ représente, séparément ou conjointement, -O- ou -NH- ; 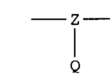
D représente un segment
Z est un radical trivalent hydrocarboné pouvant contenir un ou plusieurs hétéroatomes ;
Q est un segment polysiloxanique ;
R est un radical divalent choisi parmi les radicaux alkylènes de type aliphatique, cycloaliphatique ou aromatique.

4. Utilisation selon la revendication 3, caractérisée en ce que ledit segment Q répond à la formule générale (I') suivante : 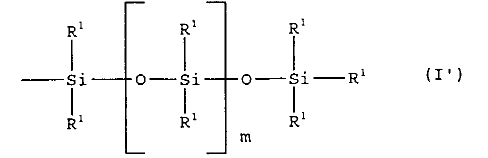 dans laquelle les radicaux R¹, identiques ou différents, sont choisis parmi d'une part les radicaux hydrocarbonés, hydrohalogénocarbonés ou perhalogénés monovalents en C₁-C₂₀ exempts d'insaturations éthyléniques et d'autre part les radicaux aromatiques ; m est un nombre entier tel que le poids moléculaire moyen mesuré en sommet de pic G.P.C. du segment polysiloxane soit compris entre 300 et 50.000.

5. Utilisation selon la revendication 4, caractérisé par le fait que les radicaux R¹ sont choisis parmi les radicaux alkyles, les radicaux cycloalkyles, les radicaux aryles , les radicaux arylalkyles.

6. Utilisation selon l'une quelconque des revendications 3 à 5, caractérisée par le fait que le radical trivalent Z est choisi parmi les radicaux 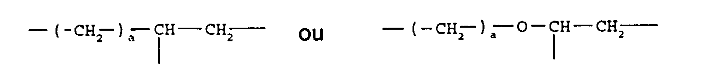 où a représente un nombre entier allant de 1 à 10.

7. Utilisation selon l'une quelconque des revendications 2 à 6, caractérisée par le fait que la chaîne comporte la répétition de séquences polysiloxanes -[-L-]- répondant à la formule générale (II) suivante : 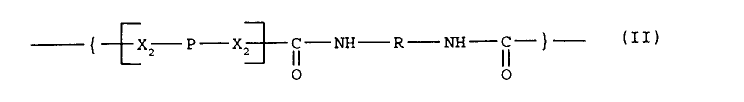 dans laquelle :
P est un segment polysiloxane,
X₂ représente, séparement ou conjointement -O- ou -NH-,
R a la même signification indiquée dans la revendication 3.

8. Utilisation selon la revendication 7, caractérisée par le fait que le segment polysiloxane P a pour formule générale : 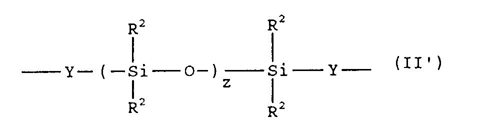 dans laquelle :
Y est un radical divalent hydrocarboné pouvant contenir un ou plusieurs hétéroatomes ;
R² a les mêmes significations que R¹ indiquées dans les revendications 4 et 5 ;
z est un nombre entier tel que le poids moléculaire moyen mesuré en sommet de pic G.P.C. du segment polysiloxane soit compris entre 300 et 50.000.

9. Utilisation selon la revendication 8, caractérisée par le fait que le polysiloxane P répond à la formule suivante : 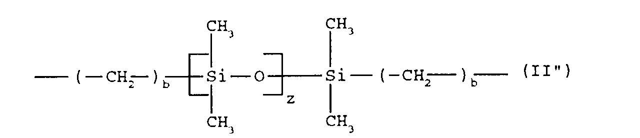 dans laquelle b représente un nombre entier allant de 1 à 10 et z est un nombre entier tel que le poids moléculaire moyen mesuré en sommet de pic G.P.C. du segment polysiloxane soit compris entre 300 et 50.000.

10. Utilisation selon l'une quelconque des revendications 1 à 9, caractérisée par le fait que la chaîne est constituée par la répétition d'une séquence -[-N-]- de polyuréthane et/ou de polyurée répondant à la formule (III) générale suivante : 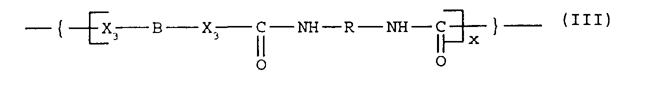 dans laquelle :
X₃ représente, séparément ou conjointement, -O- ou -NH- ;
R a la même signification indiquée dans la revendication 3 ;
x est un nombre entier allant de 1 à 10 ;
les radicaux B, identiques ou différents, sont des radicaux divalents hydrocarbonés non-ioniques ou porteurs d'une charge ionique, positive ou négative.

11. Utilisation selon l'une des revendications 10, caractérisée en ce que ledit radical B est porteur de groupement(s) présentant une ou des fonction(s) carboxylique(s) et/ou une des fonctions sulfoniques, lesdites fonctions carboxyliques et/ou sulfoniques étant neutralisées partiellement ou totalement par une base minérale ou organique.

12. Utilisation selon la revendication 11, caractérisée en ce que le radical B répond à la formule (IV) : 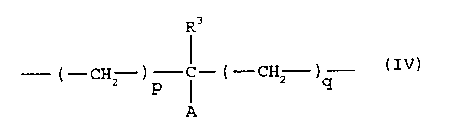 dans laquelle R³ représente un radical alkyle, linéaire ou ramifié, en C₁-C₃, A une fonction acide carboxylique (-COOH) ou acide sulfonique (-SO₃H) ou un sel desdites fonctions acides, et p et q, qui peuvent être identiques ou différents, des nombres entiers allant de 1 et 5.

13. Utilisation selon la revendication 12, caractérisée en ce que le radical B répond à la formule (IV') : 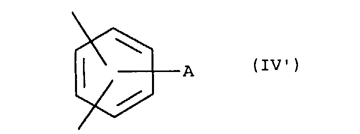 dans laquelle A a la signification ci-dessus.

14. Utilisation selon la revendication 10, caractérisé en ce que ledit radical B est porteur de groupements amines tertiaires, lesdites amines tertiaires étant, partiellement ou totalement, soit neutralisées par une base minérale ou organique, soit quaternisées, soit bétaïnisées.

15. Utilisation selon la revendication 14, caractérisée en ce que le radical B répond à la formule (V) suivante : 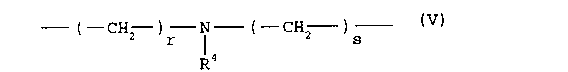 dans laquelle R⁴ représente un radical alkyle, linéaire ou ramifié, en C₁-C₄, et r et s deux nombres entiers, identiques ou différents, qui peuvent être compris entre 1 et 10.

16. Utilisation selon la revendication 15, caractérisée en ce que, sous forme neutralisée, quaternisée ou bétaïnisées le radical B répond à la formule (V') suivante : 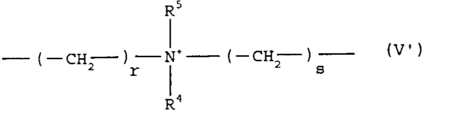 formule dans laquelle R⁴ a la signification ci-dessus, et R⁵ représente soit l'hydrogène (neutralisation) soit un radical alkyle, linéaire ou ramifié, en C₁-C₁₀ ou un cycle aromatique (quaternisation), soit un radical alkyle, linéaire ou ramifié en C₁-C₁₀ porteur d'un groupe carboxylate ou sulfonate (bétaïnisation).

17. Utilisation selon la revendication 10, caractérisée par le fait que ledit radical B est porteur de groupes non-ioniques choisis parmi les motifs apportés par les α, ω-diols; les α, ω-diamines et les alcoolamines aliphatiques, cycloaliphatiques ou aromatiques.

18. Utilisation selon l'une quelconque des revendications 10 à 18, caractérisé par le fait qu'il comprend des séquences -[-N-]- dans lesquelles figurent un segment B non-ionique et des séquences -[-N-]- dans lesquelles figurent un segment B cationique ou anionique.

19. Utilisation selon l'une quelconque des revendications 1 à 18, caractérisée par le fait que la chaîne est constituée par la répétition d'une séquence -[-G-]- polyuréthane et/ou polyurée comportant des oligomères de polymère organique répondant à la formule suivante : 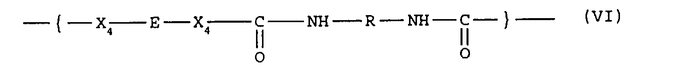 dans laquelle :
X₄ représente, séparément ou conjointement, -O- ou -NH- ;
R a la même signification indiquée dans la revendication 3 ;
E est un oligomère d'un polymère organique choisi parmi les polyéthers, les polyesters, les polyester-amides, les polyamides, de poids moléculaire moyen mesuré en sommet de pic G.P.C., compris entre 300 et 70.000 et pouvant contenir des greffons sur la chaîne ou des segments dans la chaîne, porteurs d'une charge ionique, positive ou négative.

20. Utilisation selon l'une quelconque des revendications 3 à 19, caractérisée en ce que le radical R est choisi parmi les radicaux de formules suivantes : 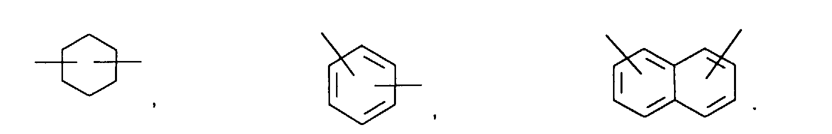 ou 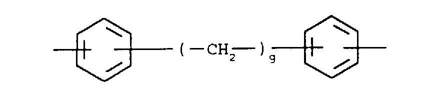 ou 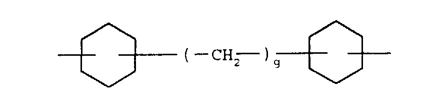 ou
-(-CH₂-)ₕ-
dans lesquelles g est un nombre entier compris entre 0 et 3, et h un nombre entier compris entre 1 et 20, de préférence compris entre 2 et 12.

21. Utilisation dans et pour la fabrication d'une composition cosmétique ou dermatologique d'un pseudo-latex susceptible d'être obtenu par un procédé de préparation consistant à utiliser un polycondensat multiséquencé dont la chaîne est constituée par la répétition d'au moins une séquence -[-M-]- et d'au moins une séquence de polyuréthane et/ou polyurée -[-N-]- et/ou -[-G-]- comportant des groupes cationisables ou anionisables, tel que défini dans l'une quelconque des revendications 2 à 20, à neutraliser, partiellement ou totalement, les groupes cationisables ou anionisables dans un solvant ou un mélange de solvants organiques présentant un point d'ébullition inférieur à celui de l'eau, miscibles ou partiellement miscibles à l'eau ; que l'on disperse la solution ainsi obtenue dans une solution aqueuse ; puis que l'on élimine le ou les solvants organiques.

22. Utilisation selon la revendication 21, caractérisée en ce que le taux de neutralisation varie de 10 à 100 % et de préférence de 20 à 100 %.

23. Composition cosmétique ou dermatologique caractérisée par le fait qu'elle contient dans un milieu cosmétiquement acceptable au moins un polycondensat séquencé défini selon l'une quelconque des revendications 1 à 20.

24. Composition selon la revendication 23, caractérisée par le fait qu'elle se présente sous forme de pseudo-latex tel que défini dans l'une quelconque des revendications 21 et 22.

25. Composition selon la revendication 23, caractérisée par le fait qu'elle se présente sous forme de solution du polycondensat séquencé polyuréthane et/ou polyurée à greffons siliconés dans un solvant organique; sous forme d'«émulsion» ou de dispersion dans l'eau d'une solution dudit polycondensat dans un solvant organique ; ou bien sous forme de solution hydroorganique dudit polycondensat.

26. Composition selon l'une quelquonque des revendications 23 à 25, caractérisée par le fait qu'elle contient de 0,5 à 50% en poids de polycondensat séquencé polyuréthane et/ou polyurée à greffons siliconés ou de pseudo-latex tel que défini dans la revendication 24 par rapport au poids total de la composition.

27. Composition selon l'une quelquonque des revendications 23 à 27, caractérisée par le fait qu'elle contient des additifs choisis parmi les corps gras, des solvants organiques, des agents épaississants, des adoucissants, des agents anti-mousse, des agents hydratants, des humectants, des agents traitants, des antiperspirants, des agents alcanisants, des colorants, des pigments, des parfums, des conservateurs, des agents propulseurs, des silicones et/ou des polymères organiques anioniques, non-ioniques ou amphotères compatibles avec des polyuréthanes ou des polyurées, des filtres solaires UV-A et/ou UV-B.

28. Produit pour le lavage et/ou le soin et/ou le traitement des cheveux, caractérisé par le fait qu'il est constitué par une composition selon l'une quelconque des revendications 23 à 27.

29. Produit pour le maquillage, caractérisée par le fait qu'il est constitué par une composition selon l'une quelconque des revendications 23 à 27.

30. Produit pour le soin de la peau, caractérisée par le fait qu'il est constitué par une composition selon l'une quelconque des revendications 23 à 27.

31. Utilisation d'un polycondensat selon l'une quelconque des revendications 1 à 20 comme agent filmogène ou comme additif d'agent filmogène dans une composition cosmétique ou dermatologique.

32. Utilisation d'un pseudo-latex selon l'une quelconque des revendications 21 et 22 comme agent filmogène ou comme additif d'agent filmogène dans une composition cosmétique ou dermatologique.

33. Procédé de traitement cosmétique des matières kératiniques, caractérisé en ce qu'il consiste à appliquer sur ces dernières, une composition selon l'une quelconque des revendications 23 à 27.

34. Polycondensat multiséquencé polyuréthane et/ou polyurée à greffons polysiloxanes, caractérisé par le fait que la chaîne est constituée par la répétition d'au moins une séquence polyuréthane et/ou polyurée -[-M-]- comportant un greffon polysiloxane et également :
(i) par la répétition d'une séquence de polyuréthane et/ou polyurée -[-N-]-répondant à la formule (III) générale suivante : 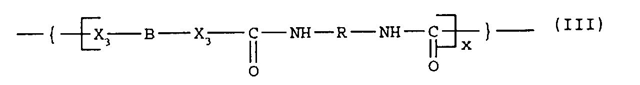 dans laquelle :
X₃ représente, séparément ou conjointement, -O- ou -NH- ;
R est un radical divalent choisi parmi les radicaux alkylènes de type aliphatique, cycloaliphatique ou aromatique ;
x est un nombre entier allant de 1 à 10 ;
les radicaux B, identiques ou différents, peuvent désigner :
(1) des radicaux divalents hydrocarbonés porteurs de groupes non-ioniques choisis parmi les motifs apportés par les α, ω-diols; les α, ω-diamines et les alcoolamines aliphatiques, cycloaliphatiques ou aromatiques ;
(2) des radicaux divalents hydrocarbonés porteurs de groupes anioniques présentant une ou des fonction(s) carboxylique(s) et/ou une des fonctions sulfoniques, lesdites fonctions carboxyliques et/ou sulfoniques étant neutralisées partiellement ou totalement par une base minérale ou organique ;
(3) des radicaux divalents hydrocarbonés porteurs de groupes porteurs de groupements amines tertiaires, lesdites amines tertiaires étant, partiellement ou totalement, soit neutralisées par une base minérale ou organique, soit quaternisées, soit bétaïnisées ; et/ou
(ii) par la répétition d'une séquence de polyuréthane et/ou polyurée -[-G-]- comportant des oligomères de polymères organiques de poids moléculaire moyen mesuré en sommet de pic G.P.C., compris entre 300 et 70.000 et (iii) par la répétition d'une séquence polysiloxane -[-L-]- répondant à la formule générale (II) suivante : 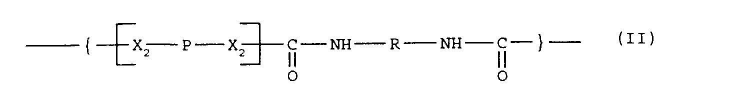 dans laquelle :
P est un segment polysiloxane,
X₂ représente, séparement ou conjointement -O- ou -NH-,
R est un radical divalent choisi parmi les radicaux alkylènes de type aliphatique, cycloaliphatique ou aromatique.

35. Polycondensat selon la revendication 34, caractérisé par le fait que le segment polysiloxane P a pour formule générale : 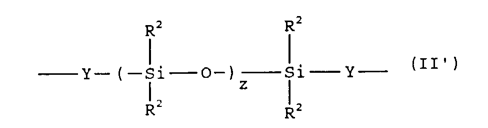 dans laquelle :
Y est un radical divalent hydrocarboné pouvant contenir un ou plusieurs hétéroatomes ;
R² a les mêmes significations que R¹ indiquées dans les revendications 4 et 5 ;
z est un nombre entier tel que le poids moléculaire moyen mesuré en sommet de pic G.P.C. du segment polysiloxane soit compris entre 300 et 50.000.

36. Polycondensat selon la revendication 35, caractérisé par le fait que le polysiloxane P répond à la formule suivante : 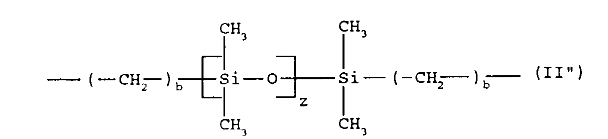 dans laquelle b représente un nombre entier allant de 1 à 10 et z est un nombre entier tel que le poids moléculaire moyen mesuré en sommet de pic G.P.C. du segment polysiloxane soit compris entre 300 et 50.000.

37. Polycondensat selon la revendication 34, caractérisé en ce que le radical B répond à la formule (IV) : 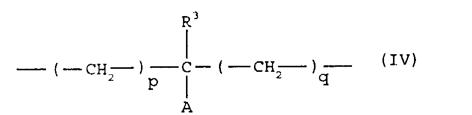 dans laquelle R³ représente un radical alkyle, linéaire ou ramifié, en C₁-C₃, A une fonction acide carboxylique (-COOH) ou acide sulfonique (-SO₃H) ou un sel desdites fonctions acides, et p et q, qui peuvent être identiques ou différents, des nombres entiers compris entre 1 et 5.

38. Polycondensat selon la revendication 37, caractérisé en ce que le radical B répond à la formule (IV') : 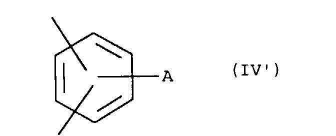 dans laquelle A a la signification ci-dessus.

39. Polycondensat, selon la revendication 34, caractérisé en ce que le radical B répond à la formule (V) suivante : 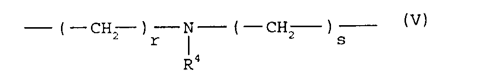 dans laquelle R⁴ représente un radical alkyle, linéaire ou ramifié, en C₁-C₄, et r et s deux nombres entiers, identiques ou différents, qui peuvent être compris entre 1 et 10.

40. Polycondensat selon la revendication 34, caractérisé en ce que, sous forme neutralisée, quaternisée ou bétaïnisée, le radical B répond à la formule (V') suivante : 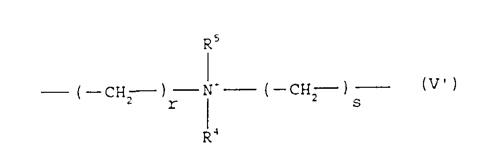 formule dans laquelle R⁴ a la signification ci-dessus, et R⁵ représente soit l'hydrogène (neutralisation) soit un radical alkyle, linéaire ou ramifié, en C₁-C₁₀ ou un cycle aromatique (quaternisation), soit un radical alkyle, linéaire ou ramifié en C₁-C₁₀ porteur d'un groupe carboxylate ou sulfonate (bétaïnisation).

41. Polycondensat selon l'une quelconque des revendications 34 à 40, caractérisé par le fait qu'il comprend des séquences -[-N-]- dans lesquelles figurent un segment B non-ionique et des séquences -[-N-]- dans lesquelles figurent un segment B cationique ou anionique.

42. Polycondensat selon l'une quelconque des revendications 34 à 41, caractérisé par le fait que la chaîne est constituée par la répétition d'une séquence -[-G-]- polyuréthane et/ou polyurée comportant des oligomères de polymère organique répondant à la formule suivante : 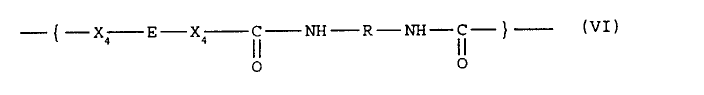 dans laquelle :
X⁴ représente, séparément ou conjointement, -O- ou -NH- ;
R est un radical divalent choisi parmi les radicaux alkylènes de type aliphatique, cycloaliphatique ou aromatique.
E est un oligomère d'un polymère organique choisi parmi les polyéthers, les polyesters, les polyester-amides, les polyamides, de poids moléculaire moyen mesuré en sommet de pic G.P.C., compris entre 300 et 70.000 et pouvant contenir des greffons sur la chaîne ou des segments dans la chaîne, porteurs d'une charge ionique, positive ou négative.

43. Polycondensat selon l'une quelconque des revendications 34 à 42, caractérisé en ce que le radical R est choisi parmi les radicaux de formules suivantes : 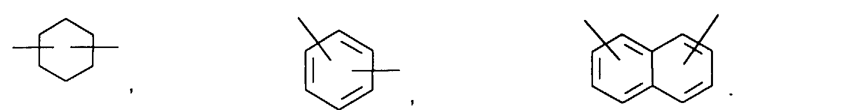 ou 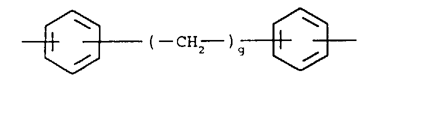 ou 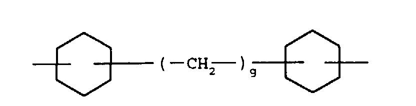 ou
-(-CH₂-)ₕ-
dans lesquelles g est un nombre entier compris entre 0 et 3, et h un nombre entier compris entre 1 et 20, de préférence compris entre 2 et 12.

44. Procédé de préparation d'un pseudo-latex caractérisé par le fait que l'on utilise un polycondensat multiséquencé dont la chaîne est constituée par la répétition d'au moins une séquence -[-M-]- et d'au moins une séquence de polyuréthane et/ou polyurée -[-N-]- et/ou -[-G-]- comportant des groupes cationisables ou anionisables, tel que défini dans l'une quelconque des revendications 34 à 43, que l'on neutralise, partiellement ou totalement, les groupes cationisables ou anionisables dans un solvant organique ou un mélange de solvants organiques présentant un point d'ébullition inférieur à celui de l'eau, miscibles ou partiellement miscibles à l'eau ; que l'on disperse la solution ainsi obtenue dans une solution aqueuse ; puis que l'on élimine le ou les solvants organiques.

45. Procédé selon la revendication 44, caractérisé en ce que le taux de neutralisation varie de 10 à 100 % et de préférence de 20 à 100 %.

46. Pseudo-latex susceptible d'être obtenu par le procédé de la revendication 44 ou 45.

## Patentansprüche

1. Verwendung eines sequentiellen Polyurethan- und/oder Polyharnstoff-Polykondensats, das eine Kette aufweist, die aus wiederkehrenden Einheiten mindestens eines Polyurethan- und/oder Polyharnstoffblocks -[-M-]- besteht, welcher Polysiloxanpfropfzweige enthält, in einer kosmetischen oder dermatologischen Zusammensetzung und zur Herstellung dieser Zusammensetzung.

2. Verwendung nach Anspruch 1, dadurch gekennzeichnet, daß das Polykondensat multisequentiell ist und die Kette ferner aus wiederkehrenden Einheiten eines Polyurethanund/oder Polyharnstoffblocks -[-N-]-, der nichtionische und/oder ionische Gruppen enthält, und/oder eines Polyurethan- und/oder Polyharnstoffblocks -[-G-]-, der Oligomere von organischen Polymeren eines mittleren Molekülgewichts im Bereich von 300 bis 70 000, das im Maximum des GPC-Peaks bestimmt ist, aufweist, und/oder eines Polysiloxanblocks -[-L-]- besteht.

3. Verwendung nach einem der Ansprüche 1 und 2, dadurch gekennzeichnet, daß der mit einem Polysiloxan gepfropfte Polyurethan- und/oder Polyharnstoffblock -[-M-]- der folgenden allgemeinen Formel entspricht: 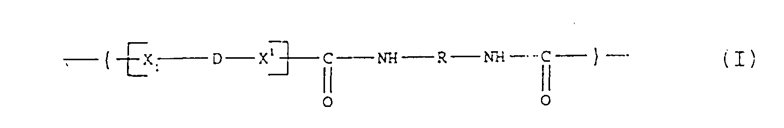 worin bedeuten:
die Gruppen X₁, unabhängig voneinander oder gleichzeitig, -O- oder -NH-;
D eine Einheit
Z eine dreiwertige Kohlenwasserstoffgruppe, die ein oder mehrere Heteroatome enthalten kann;
Q eine Polysiloxaneinheit;
R eine zweiwertige Gruppe, die unter den aliphatischen cycloaliphatischen oder aromatischen Alkylengruppen ausgewählt ist.

4. Verwendung nach Anspruch 3, dadurch gekennzeichnet, daß die Einheit Q der folgenden allgemeinen Formel (I') entspricht: 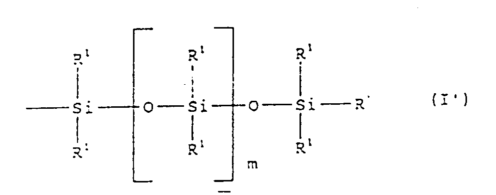 worin die Gruppen R¹, die identisch oder voneinander verschieden sind, unter den einwertigen Kohlenwasserstoffgruppen, halogenhaltigen Kohlenwasserstoffgruppen oder perhalogenierten Gruppen mit 1 bis 20 Kohlenstoffatomen, die keine ethylenischen Doppelbindungen enthalten, und den aromatischen Gruppen ausgewählt sind, und m eine ganze Zahl bedeutet, die so gewählt ist, daß das mittlere Molekülgewicht der Polysiloxaneinheit, bestimmt am Maximum des GPC-Peaks, im Bereich von 300 bis 50 000 liegt.

5. Verwendung nach Anspruch 4, dadurch gekennzeichnet, daß die Gruppen R¹ unter den Alkylgruppen, Cycloalkylgruppen, Arylgruppen und Arylalkylgruppen ausgewählt sind.

6. Verwendung nach einem der Ansprüche 3 bis 5, dadurch gekennzeichnet, daß die dreiwertige Gruppe Z unter den folgenden Gruppen ausgewählt ist: 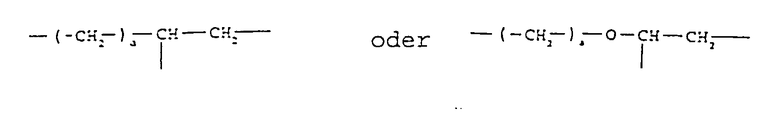 worin a eine ganze Zahl im Bereich von 1 bis 10 bedeutet.

7. Verwendung nach einem der Ansprüche 2 bis 6, dadurch gekennzeichnet, daß die Kette wiederkehrende Einheiten von Polysiloxanblöcken -[-L-]- enthält, die der folgenden allgemeinen Formel (II) entsprechen: 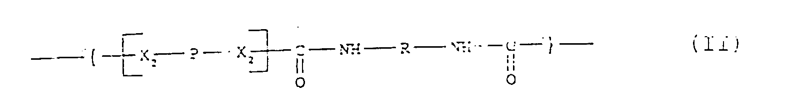 worin bedeuten:
P eine Polysiloxaneinheit,
die Gruppen X₂, unabhängig voneinander oder gleichzeitig, -O- oder -NH-,
R die in Anspruch 3 angegebene Bedeutung.

8. Verwendung nach Anspruch 7, dadurch gekennzeichnet, daß die Polysiloxaneinheit P die allgemeine Formel aufweist: 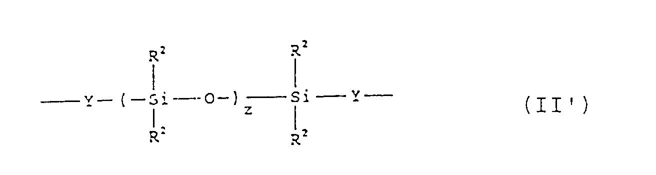 worin bedeuten:
Y eine zweiwertige Kohlenwasserstoffgruppe, die ein oder mehrere Heteroatome enthalten kann;
die Gruppen R² die in den Ansprüchen 4 und 5 für die Gruppen R¹ angegebenen Bedeutungen;
z eine ganze Zahl, die so ausgewählt ist, daß das mittlere Molekülgewicht der Polysiloxaneinheit, bestimmt am Maximum des GPC-Peaks, im Bereich von 300 bis 50 000 liegt.

9. Verwendung nach Anspruch 8, dadurch gekennzeichnet, daß das Polysiloxan P der folgenden Formel entspricht: 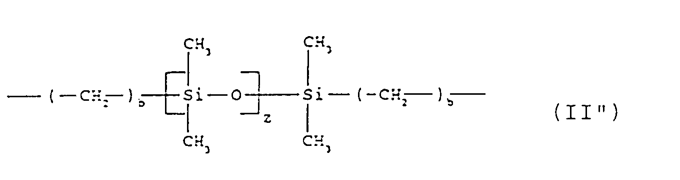 worin b eine ganze Zahl im Bereich von 1 bis 10, und z eine ganze Zahl bedeutet, die so ausgewählt ist, daß das mittlere Molekülgewicht der Polysiloxaneinheit, bestimmt am Maximum des GPC-Peaks, im Bereich von 300 bis 50 000 liegt.

10. Verwendung nach einem der Ansprüche 1 bis 9, dadurch gekennzeichnet, daß die Kette aus wiederkehrenden Einheiten eines Polyurethan- und/oder Polyharnstoffblocks -[-N-]- besteht, der der folgenden allgemeinen Formel (III) entspricht: 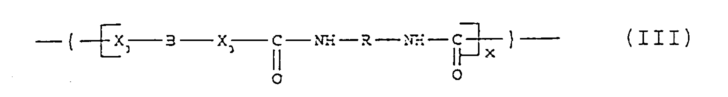 worin bedeuten:
die Gruppen X₃, unabhängig voneinander oder gleichzeitig, -O- oder -NH-;
R die in Anspruch 3 angegebene Bedeutung;
x eine ganze Zahl im Bereich von 1 bis 10;
die Gruppen B, die identisch oder voneinander verschieden sind, zweiwertige nichtionische Kohlenwasserstoffgruppen oder Kohlenwasserstoffgruppen, die eine positive oder negative Ladung aufweisen.

11. Verwendung nach Anspruch 10, dadurch gekennzeichnet, daß die Gruppe B eine Gruppe oder mehrere Gruppen aufweist, welche eine oder mehrere Carbonsäuregruppen und/oder eine oder mehrere Sulfonsäuregruppen tragen, wobei die Carboxy- und/oder Sulfonsäuregruppen vollständig oder teilweise mit einer anorganischen oder organischen Base neutralisiert sind.

12. Verwendung nach Anspruch 11, dadurch gekennzeichnet, daß die Gruppe B der folgenden Formel (IV) entspricht: 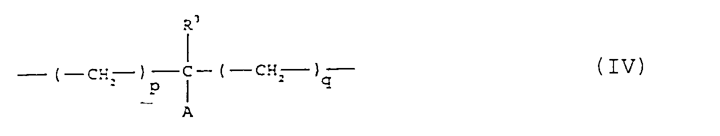 worin R³ eine geradkettige oder verzweigte C₁₋₃-Alkylgruppe, A eine Carbonsäuregruppe (-COOH) oder Sulfonsäuregruppe (-SO₃H), oder ein Salz der beiden Säuregruppen bedeutet, und p und q, die identisch oder voneinander verschieden sein können, ganze Zahlen im Bereich von 1 bis 5 sind.

13. Verwendung nach Anspruch 12, dadurch gekennzeichnet, daß die Gruppe B der folgenden Formel (IV') entspricht: 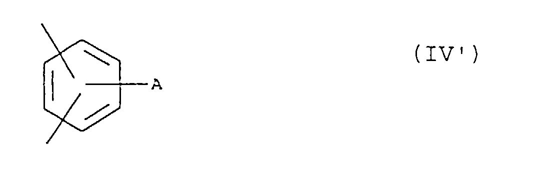 worin A die oben angegebene Bedeutung aufweist.

14. Verwendung nach Anspruch 10, dadurch gekennzeichnet, daß die Gruppe B tertiäre Aminogruppen aufweist, wobei die tertiären Aminogruppen vollständig oder teilweise mit einer anorganischen oder organischen Base neutralisiert sind, quaternisiert sind oder als Zwitterionen vorliegen.

15. Verwendung nach Anspruch 14, dadurch gekennzeichnet, daß die Gruppe B der folgenden Formel (V) entspricht: 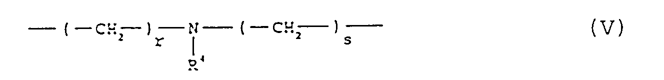 worin R⁴ eine geradkettige oder verzweigte C₁₋₄-Alkylgruppe bedeutet, und r und s zwei ganze Zahlen sind, die identisch oder voneinander verschieden sind und im Bereich von 1 bis 10 liegen können.

16. Verwendung nach Anspruch 15, dadurch gekennzeichnet, daß die Gruppe B in neutralisierter oder quaternisierter Form oder als Zwitterion vorliegend der folgenden Formel (V') entspricht: 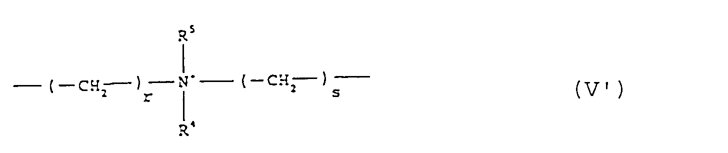 wobei in der Formel R⁴ die oben angegebene Bedeutung aufweist und R⁵ entweder Wasserstoff (Neutralisation) oder eine geradkettige oder verzweigte C₁₋₁₀-Alkylgruppe oder einen aromatischen Ring (Quaternisierung) oder eine geradkettige oder verzweigte C₁₋₁₀-Alkylgruppe, die eine Carboxy- oder Sulfonatgruppe (Zwitterion) trägt, bedeutet.

17. Verwendung nach Anspruch 10, dadurch gekennzeichnet, daß die Gruppe B nichtionische Gruppen aufweist, die unter den Einheiten ausgewählt sind, die von aliphatischen, cycloaliphatischen oder aromatischen α,ω-Diolen, α,ω-Diaminen und Alkoholaminen eingebracht werden.

18. Verwendung nach einem der Ansprüche 10 bis 17, dadurch gekennzeichnet, daß Blöcke -[-N-]- enthalten sind, worin nichtionische Einheiten B vorhanden sind, und Blöcke -[-N-]-, worin kationische oder anionische Einheiten B enthalten sind.

19. Verwendung nach einem der Ansprüche 1 bis 18, dadurch gekennzeichnet, daß die Kette aus wiederkehrenden Einheiten eines Polyurethan- und/oder Polyharnstoffblocks -[-G-]- besteht, der Oligomere eines organischen Polymers enthält und der folgenden Formel entspricht: 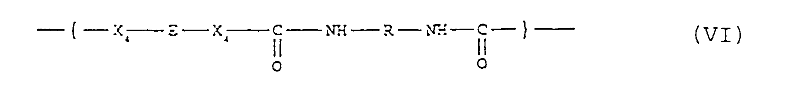 worin bedeuten:
die Gruppen X₄, unabhängig voneinander oder gleichzeitig, -O- oder -NH-;
R die in Anspruch 3 angegebene Bedeutung;
E ein Oligomer eines organischen Polymers, das unter den Polyethern, Polyestern, Polyesteramiden und Poly amiden ausgewählt ist und ein mittleres Molekülgewicht aufweist, das, bestimmt am Maximum des GPC-Peaks, im Bereich von 300 bis 70 000 liegt, wobei an der Kette Pfropfzweige oder in der Kette Einheiten enthalten sein können, die positiv oder negativ geladen sind.

20. Verwendung nach einem der Ansprüche 3 bis 19, dadurch gekennzeichnet, daß die Gruppe R unter den folgenden Gruppen ausgewählt ist: 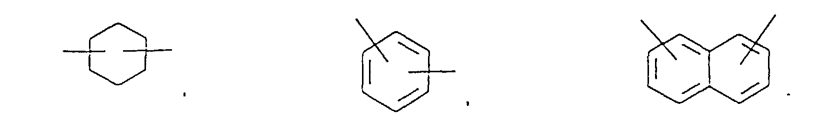 oder 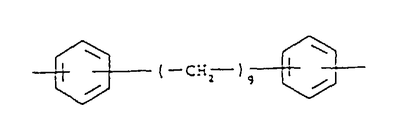 oder 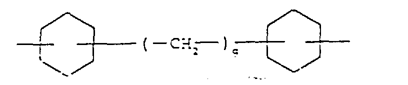 oder
-(-CH₂-)ₕ-
worin g eine ganze Zahl im Bereich von Null bis 3, und h eine ganze Zahl im Bereich von 1 bis 20 und vorzugsweise 2 bis 12 bedeutet.

21. Verwendung eines Pseudo-Latex in einer kosmetischen oder dermatologischen Zusammensetzung oder zu deren Herstellung, der nach einem Herstellungsverfahren erhalten werden kann, das darin besteht, ein multisequentielles Polykondensat nach einem der Ansprüche 2 bis 20 zu verwenden, dessen Kette aus wiederkehrenden Einheiten mindestens eines Blockes -[-M-]- und mindestens eines Polyurethan- und/oder Polyharnstoffblocks -[-N-]-und/oder -[-G-]-, die Gruppen aufweist, die in Kationen oder Anionen umgewandelt werden können, besteht, die in Kationen oder Anionen umwandelbaren Gruppen in einem organischen Lösungsmittel oder einem Gemisch von organischen Lösungsmitteln vollständig oder teilweise zu neutralisieren, welche einen Siedepunkt unter dem Siedepunkt des Wassers aufweisen und mit Wasser mischbar oder teilweise mischbar sind, wobei die so hergestellte Lösung in einer wäßrigen Lösung dispergiert und anschließend das oder die organischen Lösungsmittel entfernt werden.

22. Verwendung nach Anspruch 21, dadurch gekennzeichnet, daß der Neutralisationsgrad im Bereich von 10 bis 100 % und vorzugsweise von 20 bis 100 % liegt.

23. Kosmetische oder dermatologische Zusammensetzung, dadurch gekennzeichnet, daß sie in einem kosmetisch akzeptablen Medium mindestens ein sequentielles Polykondensat nach einem der Ansprüche 1 bis 20 enthält.

24. Zusammensetzung nach Anspruch 23, dadurch gekennzeichnet, daß sie in Form eines Pseudo-Latex nach einem der Ansprüche 21 bis 22 vorliegt.

25. Zusammensetzung nach Anspruch 23, dadurch gekennzeichnet, daß sie in Form einer Lösung des sequentiellen Polyurethan- und/oder Polyharnstoff-Polykondensats mit Siliconpfropfzweigen in einem organischen Lösungsmittel, in Form einer «Emulsion» oder Dispersion in Wasser einer Lösung des Polykondensats in einem organischen Lösungsmittel oder auch in Form einer wäßrig-organischen Lösung des Polykondensats vorliegt.

26. Zusammensetzung nach einem der Ansprüche 23 bis 25, dadurch gekennzeichnet, daß sie 0,5 bis 50 Gew.-% sequentielles Polyurethan- und/oder Polyharnstoff-Polykondensat mit Siliconpfropfzweigen oder Pseudo-Latex nach Anspruch 24, bezogen auf das Gesamtgewicht der Zusammensetzung, enthält.

27. Zusammensetzung nach einem der Ansprüche 23 bis 26, dadurch gekennzeichnet, daß sie Hilfsstoffe enthält, die unter den Fettsubstanzen, organischen Lösungsmitteln, Verdickungsmitteln, reizlindernden Mitteln, Antischaummitteln, HydratisierungsmitteIn, Feuchthaltemitteln, Behandlungsmitteln, Antitranspirantien, Mitteln zum Alkalischmachen, Färbemitteln, Pigmenten, Parfums, Konservierungsmitteln, Treibmitteln, Siliconen und/oder anionischen, nichtionischen oder amphoteren organischen Polymeren, die mit den Polyurethanen oder Polyharnstoffen kompatibel sind, und UV-A- und/oder UV-B-Sonnenschutzfiltern ausgewählt sind.

28. Produkt zum Waschen und/oder zur Pflege und/oder zur Behandlung der Haare, dadurch gekennzeichnet, daß es aus einer Zusammensetzung nach einem der Ansprüche 23 bis 27 besteht.

29. Produkt zum Schminken, dadurch gekennzeichnet, daß es aus einer Zusammensetzung nach einem der Ansprüche 23 bis 27 besteht.

30. Produkt zur Pflege der Haut, dadurch gekennzeichnet, daß es aus einer Zusammensetzung nach einem der Ansprüche 23 bis 27 besteht.

31. Verwendung eines Polykondensats nach einem der Ansprüche 1 bis 20 als filmbildendes Mittel oder als zusätzliches filmbildendes Mittel in einer kosmetischen oder dermatologischen Zusammensetzung.

32. Verwendung eines Pseudo-Latex nach einem der Ansprüche 21 und 22 als filmbildendes Mittel oder zusätzliches filmbildendes Mittel in einer kosmetischen oder dermatologischen Zusammensetzung.

33. Verfahren zur kosmetischen Behandlung von Keratinmaterialien, dadurch gekennzeichnet, daß es darin besteht, auf diese eine Zusammensetzung nach einem der Ansprüche 23 bis 27 aufzutragen.

34. Multisequentielles Polyurethan- und/oder PolyharnstoffPolykondensat mit Polysiloxanpfropfzweigen, dadurch gekennzeichnet, daß die Kette aus wiederkehrenden Einheiten mindestens eines Polyurethan- und/oder Polyharnstoffblocks -[-M-]- mit Polysiloxanpfropfzweigen besteht und ferner enthalten sind:
(i) wiederkehrende Einheiten eines Polyurethan- und/oder Polyharnstoffblocks -(-N-)- der folgenden allgemeinen Formel (III) 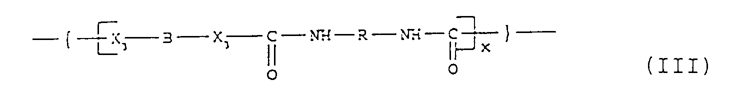 worin bedeuten:
die Gruppen X₃, unabhängig voneinander oder gleichzeitig, -O- oder -NH-;
R eine zweiwertige Gruppe, die unter den aliphatischen, cycloaliphatischen oder aromatischen Alkylengruppen ausgewählt ist;
x eine ganze Zahl im Bereich von 1 bis 10;
die Gruppen B die identisch oder voneinander verschieden sind:
(1) zweiwertige Kohlenwasserstoffgruppen, die nichtionische Gruppen tragen, die unter den Einheiten ausgewählt sind, die von aliphatischen cycloaliphatischen oder aromatischen α, ω-Diolen, α, ω-Diaminen und Alkoholaminen eingebracht werden;
(2) zweiwertige Kohlenwasserstoffgruppen, die anionische Gruppen tragen, welche eine oder mehrere Carboxy- und/oder eine oder mehrere Sulfonsäuregruppen aufweisen, wobei die Carboxy- und/oder Sulfonsäuregruppen vollständig oder teilweise mit einer anorganischen oder organischen Base neutralisiert sind;
(3) zweiwertige Kohlenwasserstoffgruppen, die Gruppen aufweisen, die tertiäre Aminogruppen tragen, wobei die tertiären Aminogruppen vollständig oder teilweise entweder mit einer organischen oder anorganischen Base neutralisiert oder quaternisiert wurden oder als Zwitterionen vorliegen und/oder
(ii) wiederkehrende Einheiten eines Polyurethan - und/oder Polyharnstoffblocks -[-G-]-, der Oligomere von organischen Polymeren eines mittleren Molekülgewichts, bestimmt am Maximum des GPC-Peaks, im Bereich von 300 bis 70 000 aufweist und (iii) wiederkehrende Einheiten eines Polysiloxanblocks -[-L-]- der folgenden allgemeinen Formel (II) : 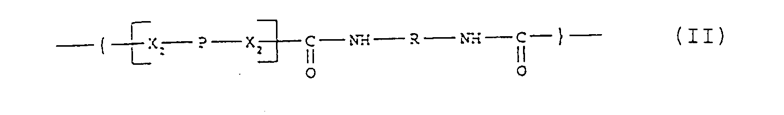 worin bedeuten
P eine Polysiloxaneinheit,
die Gruppen X₂, unabhängig voneinander oder gleichzeitig, -O- oder -NH-,
R eine zweiwertige Gruppe, die unter den aliphatischen, cycloaliphatischen oder aromatischen Alkylengruppen ausgewählt ist.

35. Polykondensat nach Anspruch 34, dadurch gekennzeichnet, daß die Polysiloxaneinheit P der allgemeinen Formel entspricht: 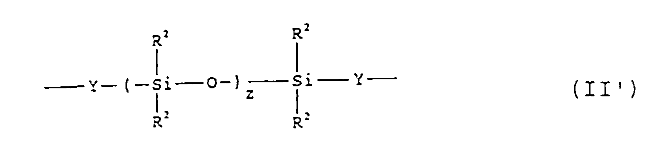 worin bedeuten:
Y eine zweiwertige Kohlenwasserstoffgruppe, die ein oder mehrere Heteroatome enthalten kann;
die Gruppen R² die in Anspruch 4 und 5 für die Gruppen R¹ angegebenen Bedeutungen;
z eine ganze Zahl, die so ausgewählt ist, daß das mittlere am Maximum des GPC-Peaks bestimmte Molekülgewicht der Polysiloxaneinheit im Bereich von 300 bis 50 000 liegt.

36. Polykondensat nach Anspruch 35, dadurch gekennzeichnet, daß das Polysiloxan P der folgenden Formel entspricht: 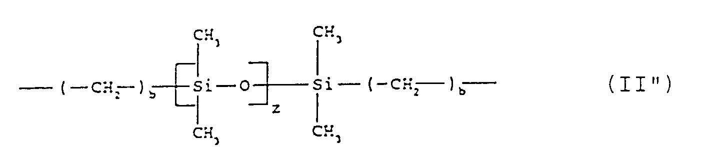 worin b eine ganze Zahl im Bereich von 1 bis 10, und z eine ganze Zahl bedeutet, die so ausgewählt ist, daß das mittlere am Maximum des GPC-Peaks bestimmte Molekülgewicht der Polysiloxaneinheit im Bereich von 300 bis 50 000 liegt.

37. Polykondensat nach Anspruch 34, dadurch gekennzeichnet, daß die Gruppe B der Formel (IV) entspricht: 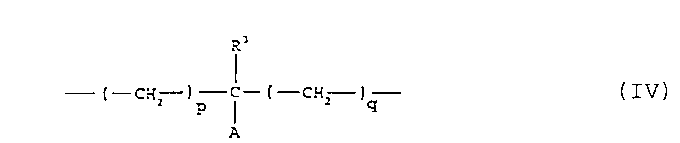 worin R³ eine geradkettige oder verzweigte C_{1,3}-Alkylgruppe, A eine Carbonsäuregruppe (-COOH) oder Sulfonsäuregruppe (-SO₃H) oder ein Salz dieser beiden Säuregruppen, und p und q, die identisch oder voneinander verschieden sein können, ganze Zahlen im Bereich von 1 bis 5 bedeuten.

38. Polykondensat nach Anspruch 37, dadurch gekennzeichnet, daß die Gruppe B der Formel (IV') entspricht: 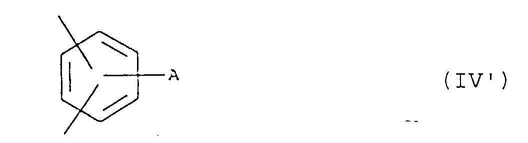 worin A die oben angegebene Bedeutung aufweist.

39. Polykondensat nach Anspruch 34, dadurch gekennzeichnet, daß die Gruppe B der folgenden Formel (V) entspricht: 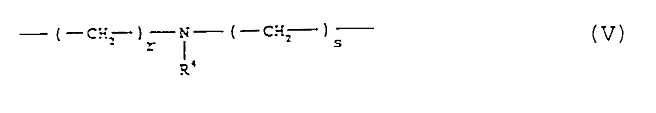 worin R⁴ eine geradkettige oder verzweigte C₁₋₄-Alkyl gruppe, und r und s zwei ganze Zahlen bedeuten, die identisch oder voneinander verschieden sind und im Bereich von 1 bis 10 liegen können.

40. Polykondensat nach Anspruch 34, dadurch gekennzeichnet, daß die Gruppe B in neutralisierter, quaternisierter oder als Zwitterion vorliegender Form der vorliegenden Formel (V') entspricht: 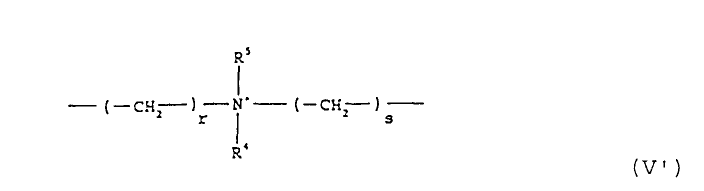 worin R⁴ die oben angegebene Bedeutung aufweist und R⁵ entweder Wasserstoff (Neutralisation) oder eine geradkettige oder verzweigte C_{1,10}-Alkylgruppe oder einen aromatischen Ring (Quaternisierung) oder eine geradkettige oder verzweigte C₁₋₁₀-Alkylgruppe bedeudet, welche eine Carboxy- oder Sulfonatgruppe (Zwitterion) trägt.

41. Polykondensat nach einem der Ansprüche 34 bis 40, dadurch gekennzeichnet, daß es Blöcke -[-N-]- mit nichtionischer Einheit B und Blöcke -[-N-]- mit kationischer oder anionischer Einheit B enthält.

42. Polykondensat nach einem der Ansprüche 34 bis 41, dadurch gekennzeichnet, daß die Kette aus wiederkehrenden Einheiten eines Polyurethan- und/oder Polyharnstoffblocks -[-G-]- mit Oligomeren eines organischen Polymers der folgenden Formel besteht: 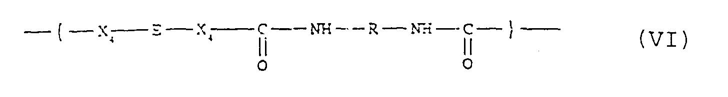 worin bedeuten:
die Gruppen X⁴, unabhängig voneinander oder gleichzeitig, -O- oder -NH-;
R eine zweiwertige Gruppe, die unter den aliphatischen cycloaliphatischen oder aromatischen Alkylengruppen ausgewählt ist;
E ein Oligomer eines organischen Polymers, das unter den Polyethern, Polyestern, Polyesteramiden und Polyamiden ausgewählt ist und ein am Maximum des GPC-Peaks bestimmtes mittleres Molekülgewicht im Bereich von 300 bis 70 000 aufweist und an der Kette Pfropfzweige oder in der Kette Einheiten enthalten kann, die eine positive oder negative Ladung tragen.

43. Polykondensat nach einem der Ansprüche 34 bis 42, dadurch gekennzeichnet, daß die Gruppe R unter den Gruppen der folgenden Formeln ausgewählt ist: 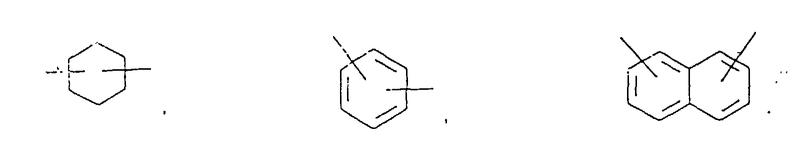 oder 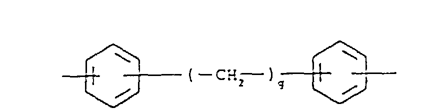 oder 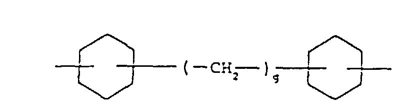 oder
-(-CH₂-)ₕ-
worin g eine ganze Zahl im Bereich von Null bis 3 und h eine ganze Zahl im Bereich von 1 bis 20 und vorzugsweise 2 bis 12 bedeutet.

44. Verfahren zur Herstellung eines Pseudo-Latex, dadurch gekennzeichnet, daß ein multisequentielles Polykondensat verwendet wird, dessen Kette aus wiederkehrenden Einheiten mindestens eines Blocks -[-M-]- und mindestens eines Polyurethan- und/oder Polyharnstoffblocks -[-N-]- und/oder -[-G-]- mit in Kationen oder Anionen umwandelbaren Blöcken nach einem der Ansprüche 34 bis 43 verwendet wird, daß die in Kationen oder Anionen umwandelbaren Gruppen vollständig oder teilweise in einem organischen Lösungsmittel oder einem Gemisch von organischen Lösungsmitteln, die einen Siedepunkt unter dem Siedepunkt von Wasser aufweisen und mit Wasser mischbar oder teilweise mischbar sind, neutralisiert werden, die so hergestellte Lösung in einer wäßrigen Lösung dispergiert wird und das oder die organischen Lösungsmittel entfernt werden.

45. Verfahren nach Anspruch 44, dadurch gekennzeichnet, daß der Neutralisationsgrad im bereich von 10 bis 100 % und vorzugsweise von 20 bis 100 % liegt.

46. Pseudo-Latex, der nach dem Verfahren des Anspruchs 44 oder 45 herstellbar ist.

## Claims

1. Use in and for the production of a cosmetic or dermatological composition of a polyurethane and/or polyurea block polycondensation product comprising a chain formed by the repetition of at least one polyurethane and/or polyurea block -[-M-]- containing a polysiloxane graft.

2. Use according to Claim 1, characterized in that the polycondensation product is multiblock and in that the chain is also formed by the repetition of a polyurethane and/or polyurea block -[-N-]- containing nonionic and/or ionic groups and/or a polyurethane and/or polyurea block -[-G-]- containing oligomers of organic polymers of average molecular weight, measured at the top of the GPC peak, of between 300 and 70,000 and/or a polysiloxane block -[-L-] -.

3. Use according to either of Claims 1 and 2, characterized in that the polysiloxane-grafted polyurethane and/or polyurea block -[-M-]- is of the following general formula: 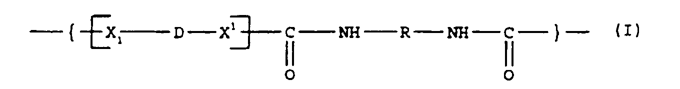 in which:
X₁ represents, separately or jointly, -O- or -NH-;
D represents a segment: 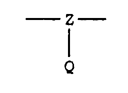
Z is a trivalent hydrocarbon radical which may contain one or more heteroatoms;
Q is a polysiloxane segment;
R is a divalent radical chosen from the alkylene radicals of aliphatic, cycloaliphatic or aromatic type.

4. Use according to Claim 3, characterized in that the said segment Q is of the following general formula (I'): 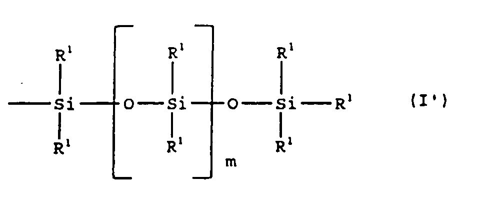 in which the radicals R¹, which are identical or different, are chosen from, on the one hand, C₁-C₂₀ monovalent hydrocarbon, halohydrocarbon or perhalogenated radicals which are free from ethylenic unsaturation, and, on the other hand, aromatic radicals, and m is an integer such that the average molecular weight, measured at the top of the GPC peak of the polysiloxane segment, is between 300 and 50,000.

5. Use according to Claim 4, characterized in that the radicals R¹ are chosen from alkyl radicals, cycloalkyl radicals, aryl radicals and arylalkyl radicals.

6. Use according to any one of Claims 3 to 5, characterized in that the trivalent radical Z is chosen from the radicals 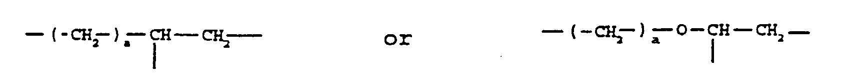 in which a represents an integer ranging from 1 to 10.

7. Use according to any one of Claims 2 to 6, characterized in that the chain comprises the repetition of polysiloxane blocks -[-L-]- of the following general formula (II): 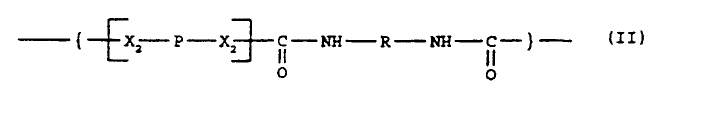 in which:
P is a polysiloxane segment,
X₂ represents, separately or jointly, -O- or -NH-, and
R has the same meaning as indicated in Claim 3.

8. Use according to Claim 7, characterized in that the polysiloxane segment P has the general formula: 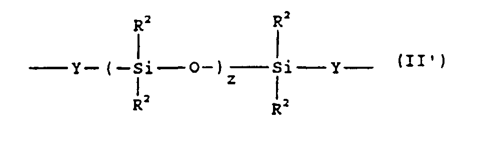 in which:
Y is a divalent hydrocarbon radical which may contain one or more heteroatoms;
R² has the same meanings as R¹ indicated in Claims 4 and 5;
z is an integer which is such that the average molecular weight, measured at the top of the GPC peak of the polysiloxane segment, is between 300 and 50,000.

9. Use according to Claim 8, characterized in that the polysiloxane P is of the following formula: 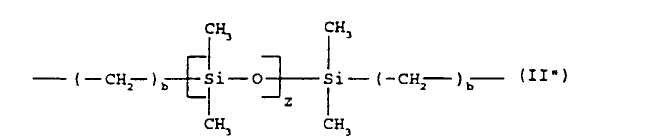 in which b represents an integer ranging from 1 to 10 and z is an integer which is such that the average molecular weight, measured at the top of the GPC peak of the polysiloxane segment, is between 300 and 50,000.

10. Use according to any one of Claims 1 to 9, characterized in that the chain is formed by the repetition of a polyurethane and/or polyurea block -[-N-]- of the following general formula (III): 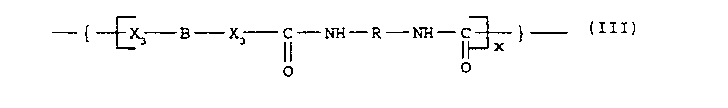 in which:
X₃ represents, separately or jointly, -O- or -NH-;
R has the same meaning as indicated in Claim 3;
x is an integer ranging from 1 to 10;
the radicals B, which are identical or different, are divalent hydrocarbon radicals which are nonionic or carry a positive or negative ionic charge.

11. Use according to Claim 10, characterized in that the said radical B carries a group or groups having one or more carboxylic functions and/or one or more sulphonic functions, the said carboxylic and/or sulphonic functions being partially or totally neutralized by an inorganic or organic base.

12. Use according to Claim 11, characterized in that the radical B is of the formula (IV): 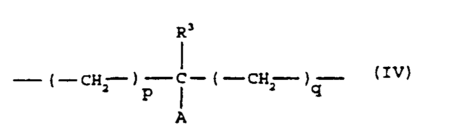 in which R³ represents a linear or branched C₁-C₃ alkyl radical, A represents a carboxylic acid function (-COOH) or sulphonic acid function (-SO₃H) or a salt of the said acid functions, and p and q, which can be identical or different, represent integers ranging from 1 to 5.

13. Use according to Claim 12, characterized in that the radical B is of the formula (IV'): 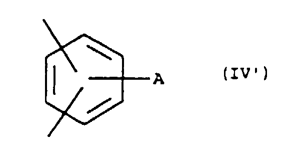 in which A has the above meaning.

14. Use according to Claim 10, characterized in that the said radical B carries tertiary amine groups, the said tertiary amines being, partially or totally, either neutralized by an inorganic or organic base or quaternized, or else betainized.

15. Use according to Claim 14, characterized in that the radical B is of the following formula (V): 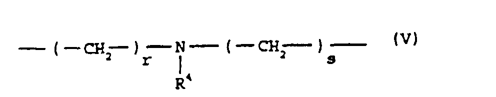 in which R⁴ represents a linear or branched C₁-C₄ alkyl radical and r and s are two identical or different integers which may be between 1 and 10.

16. Use according to Claim 15, characterized in that, in neutralized, quaternized or betainized form, the radical B is of the following formula (V'): 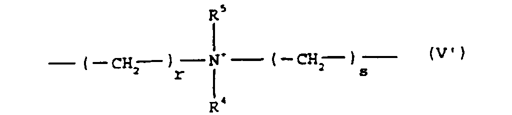 in which formula R⁴ has the above meaning and R⁵ represents either hydrogen (neutralization) or a linear or branched C₁-C₁₀ alkyl radical or an aromatic ring (quaternization), or a linear or branched C₁-C₁₀ alkyl radical which carries a carboxylate or sulphonate group (betainization).

17. Use according to Claim 10, characterized in that the said radical B carries nonionic groups chosen from the units provided by α,ω-diols, α,ω-diamines and aliphatic, cycloaliphatic or aromatic alcohol-amines.

18. Use according to any one of Claims 10 to 18, characterized in that the polycondensation product comprises blocks -[-N-]- in which there is a nonionic segment B and blocks -[-N-]- in which there is a cationic or anionic segment B.

19. Use according to any one of Claims 1 to 18, characterized in that the chain is formed by the repetition of a polyurethane and/or polyurea block -[-G-]- containing oligomers of organic polymer, of the following formula: 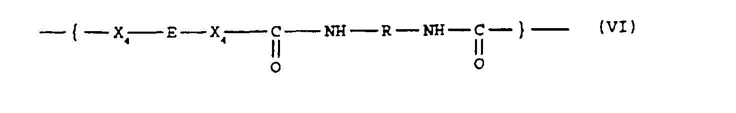 in which :
X₄ represents, separately or jointly, -O- or -NH-;
R has the same meaning as indicated in Claim 3;
E is an oligomer of an organic polymer chosen from polyethers, polyesters, polyester amides and polyamides, of average molecular weight, measured at the top of the GPC peak, of between 300 and 70,000, which can contain grafts on the chain or segments in the chain which carry a positive or negative ionic charge.

20. Use according to any one of Claims 3 to 19, characterized in that the radical R is chosen from the radicals of the following formulae: 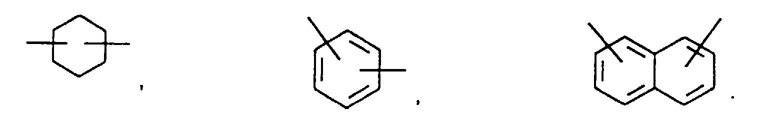 or 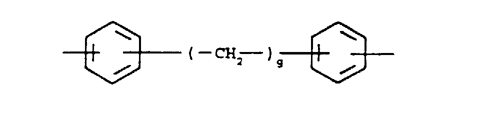 or 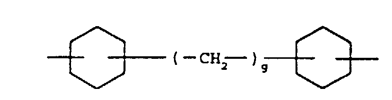 or
-(-CH₂-)ₕ-
in which g is an integer between 0 and 3, and h is an integer between 1 and 20, preferably between 2 and 12.

21. Use in and for the production of a cosmetic or dermatological composition of a pseudo-latex which can be obtained by a preparation process which consists in using a multiblock polycondensation product whose chain is formed by the repetition of at least one block -[-M-]- and at least one polyurethane and/or polyurea block -[-N-]- and/or -[-G-]- containing cationizable or anionizable groups, as is defined in any one of Claims 2 to 20, in partially or totally neutralizing the cationizable or anionizable groups in a solvent or a mixture of solvents which are organic having a boiling point less than that of water, and which are miscible or partially miscible with water; in dispersing the solution thus obtained in an aqueous solution; and then in removing the organic solvent or solvents.

22. Use according to Claim 21, characterized in that the degree of neutralization varies from 10 to 100 % and preferably from 20 to 100 %.

23. Cosmetic or dermatological composition, characterized in that it contains in a cosmetically accepable medium at least one block polycondensation product defined in accordance with any one of Claims 1 to 20.

24. Composition according to Claim 23, characterized in that it is provided in the form of a · pseudo-latex as defined in either of Claims 21 and 22.

25. Composition according to Claim 23, characterized in that it is provided in the form of a solution of the polyurethane and/or polyurea block polycondensation product containing silicone grafts in an organic solvent; in the form of an "emulsion" or dispersion in water of a solution of the said polycondensation product in an organic solvent; or else in the form of an aqueous-organic solution of the said polycondensation product.

26. Composition according to any one of Claims 23 to 25, characterized in that it contains from 0.5 to 50 % by weight of polyurethane and/or polyurea block polycondensation product containing silicone grafts or of pseudo-latex as defined in Claim 24 relative to the total weight of the composition.

27. Composition according to any one of Claims 23 to 27, characterized in that it comprises additives chosen from fats, organic solvents, thickeners, emollients, antifoams, moisturizers, humectants, treatment agents, antiperspirants, alkalifying agents, dyes, pigments, fragrances, preservatives, propellants, silicones and/or anionic, nonionic or amphoteric organic polymers which are compatible with polyurethanes or polyureas, and UV-A and/or UV-B sunscreens.

28. Product for the washing and/or care and/or treatment of hair, characterized in that it consists of a composition according to any one of Claims 23 to 27.

29. Make-up product, characterized in that it consists of a composition according to any one of Claims 23 to 27.

30. Skin care product, characterized in that it consists of a composition according to any one of Claims 23 to 27.

31. Use of a polycondensation product according to any one of Claims 1 to 20 as film-forming agent or as film-forming additive agent in a cosmetic or dermatological composition.

32. Use of a pseudo-latex according to either of Claims 21 and 22 as film-forming agent or as film-forming additive agent in a cosmetic or dermatological composition.

33. Method for the cosmetic treatment of keratinous materials, characterized in that it consists in applying to the said materials a composition according to any one of Claims 23 to 27.

34. Polyurethane and/or polyurea multiblock polycondensation product with polysiloxane grafts, characterized in that the chain is formed by the repetition of at least one polyurethane and/or polyurea block -[-M-]- containing a polysiloxane graft, and also:
(i) by the repetition of a polyurethane and/or polyurea block -[-N-]- of the following general formula (III): 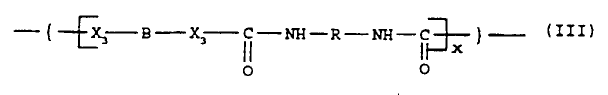 in which:
X₃ represents, separately or jointly, -O- or -NH-;
R is a divalent radical chosen from alkylene radicals of the aliphatic, cycloaliphatic or aromatic type;
x is an integer ranging from 1 to 10;
the radicals B, which are identical or different, can denote: (1) divalent hydrocarbon radicals which carry nonionic groups chosen from the units provided by the α, ω-diols; the α, ω-diamines and the aliphatic, cycloaliphatic or aromatic alcohol-amines; (2) divalent hydrocarbon radicals which carry anionic groups having one or more carboxylic functions and/or one of the sulphonic functions, the said carboxylic and/or sulphonic functions being partially or totally neutralized by an inorganic or organic base,
(3) divalent hydrocarbon radicals which carry tertiary amine groups, the said tertiary amines being, partially or totally, either neutralized by an inorganic or organic base, or quaternized, or else betainized; and/or
(ii) by the repetition of a polyurethane and/or polyurea block -[-G-]- containing oligomers of organic polymers of average molecular weight, measured at the top of the GPC peak, of between 300 and 70,000, and
(iii) by the repetition of a polysiloxane block -[-L-]- of the following general formula (II): 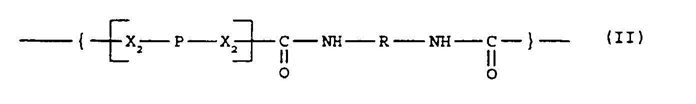 in which:
P is a polysiloxane segment,
X₂ represents, separately or jointly, -O- or -NH-, and
R is a divalent radical chosen from the alkylene radicals of aliphatic, cycloaliphatic or aromatic type.

35. Polycondensation product according to Claim 34, characterized in that the polysiloxane segment P has the general formula: 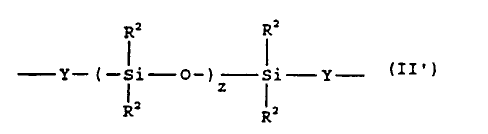 in which:
Y is a divalent hydrocarbon radical which may contain one or more heteroatoms;
R² has the same meanings as R¹ indicated in Claims 4 and 5;
z is an integer which is such that the average molecular weight, measured at the top of the GPC peak of the polysiloxane segment, is between 300 and 50,000.

36. Polycondensation product according to Claim 35, characterized in that the polysiloxane P is of the following formula: 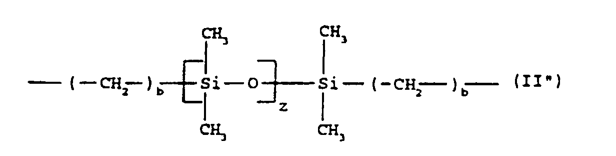 in which b represents an integer ranging from 1 to 10 and z is an integer which is such that the average molecular weight, measured at the top of the GPC peak of the polysiloxane segment, is between 300 and 50,000.

37. Polycondensation product according to Claim 34, characterized in that the radical B is of the formula (IV) : 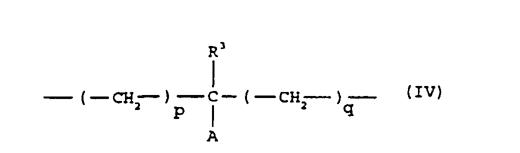 in which R³ represents a linear or branched C₁-C₃ alkyl radical, A represents a carboxylic acid function (-COOH) or sulphonic acid function (-SO₃H) or a salt of the said acid functions, and p and q, which can be identical or different, represent integers between 1 and 5.

38. Polycondensation product according to Claim 37, characterized in that the radical B is of the formula (IV') : 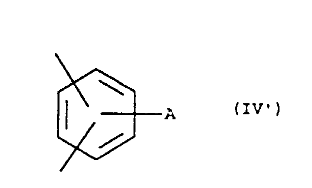 in which A has the above meaning.

39. Polycondensation product according to Claim 34, characterized in that the radical B is of the following formula (V): 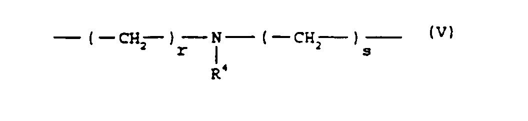 in which R⁴ represents a linear or branched C₁-C₄ alkyl radical and r and s are two identical or different integers which may be between 1 and 10.

40. Polycondensation product according to Claim 34, characterized in that, in neutralized, quaternized or betainized form, the radical B is of the following formula (V'): 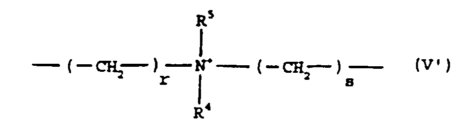 in which formula R⁴ has the above meaning and R⁵ represents either hydrogen (neutralization) or a linear or branched C₁-C₁₀ alkyl radical or an aromatic ring (quaternization), or a linear or branched C₁-C₁₀ alkyl radical which carries a carboxylate or sulphonate group (betainization).

41. Polycondensation product according to any one of Claims 34 to 40, characterized in that it comprises blocks -[-N-]- in which there is a nonionic segment B and blocks -[-N-]- in which there is a cationic or anionic segment B.

42. Polycondensation product according to any one of Claims 34 to 41, characterized in that the chain is formed by the repetition of a polyurethane and/or polyurea block -[-G-]- containing oligomers of organic polymer, of the following formula: 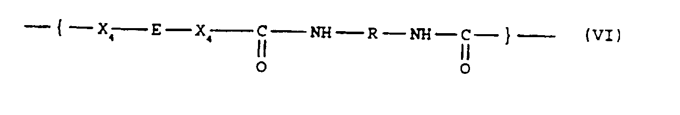 in which:
X⁴ represents, separately or jointly, -O- or -NH-;
R is a divalent radical chosen from alkylene radicals of aliphatic, cycloaliphatic or aromatic type;
E is an oligomer of an organic polymer chosen from polyethers, polyesters, polyester amides and polyamides, of average molecular weight, measured at the top of the GPC peak, of between 300 and 70,000, which can contain grafts on the chain or segments in the chain which carry a positive or negative ionic charge.

43. Polycondensation product according to any one of Claims 34 to 42, characterized in that the radical R in chosen from the radicals of the following formulae: 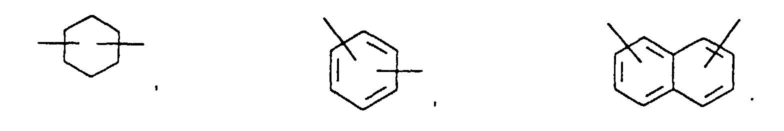 or 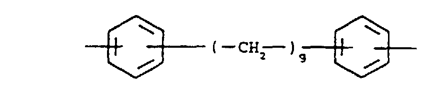 or 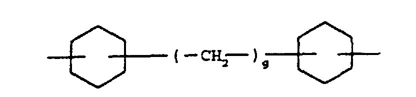 or
-(-CH₂-)ₕ-
in which g is an integer between 0 and 3, and h is an integer between 1 and 20, preferably between 2 and 12.

44. Process for the preparation of a pseudo-latex, characterized in that a multiblock polycondensation product is used whose chain is formed by the repetition of at least one block -[-M-]- and at least one polyurethane and/or polyurea block -[-N-]- and/or -[-G-]- containing cationizable or anionizable groups, as is defined in any one of Claims 34 to 43, in that the neutralization is carried out, partially or totally, of the cationizable or anionizable groups in an organic solvent or a mixture of organic solvents having a boiling point less than that of water, which are miscible or partially miscible with water; in that the solution thus obtained is dispersed in an aqueous solution; and then in that the organic solvent or solvents is or are removed.

45. Process according to Claim 44, characterized in that the degree of neutralization varies from 10 to 100 % and preferably from 20 to 100 %.

46. Pseudo-latex which can be obtained by the process of Claim 44 or 45.
